# EUROPÄISCHE PATENTSCHRIFT

(11) **EP 1 553 977 B1**
(45) Veröffentlichungstag und Bekanntmachung des Hinweises auf die Patenterteilung: **09.08.2006**
(21) Anmeldenummer: 03773451.4
(22) Anmeldetag: 19.09.2003
(51) Int. Cl.: A61K 39/21

(54) **IMPFSTOFF GEGEN INFEKTIONEN MIT ONKOVIREN, WIE DEM FELINEN LEUKOSEVIRUS DER KATZE**
VACCINE AGAINST INFECTIONS CAUSED BY ONCOVIRUSES SUCH AS THE FELINE LEUCOSIS VIRUS OF CATS
VACCIN CONTRE LES INFECTIONS PAR DES ONCOVIRUS TELS QUE LE VIRUS DE LA LEUCOSE FELINE CHEZ LE CHAT

(30) Priorität: 23.09.2002 DE 10244863
(43) Veröffentlichungstag der Anmeldung: 20.07.2005
(73) Patentinhaber: Mologen AG, 14195 Berlin (DE)
(72) Erfinder: JUNGHANS, Claas, 10551 Berlin (DE); SCHROFF, Matthias, 14057 Berlin (DE); JUHLS, Christiane, 10627 Berlin (DE); OSWALD, Detlef, 10999 Berlin (DE); LUTZ, Prof. Hans, 8455 Rüdlingen (CH)
(74) Vertreter: Omsels, Hermann-Josef
(86) Internationale Anmeldenummer: PCT/DE2003/003179
(87) Internationale Veröffentlichungsnummer: WO 2004/028562

(56) Entgegenhaltungen:
- EP-A- 0 216 564
- EP-A- 0 941 318
- WO-A-00/15824
- WO-A-03/031470
- US-B1- 6 248 582
- US-B1- 6 348 196
- TARTAGLIA J ET AL: "PROTECTION OF CATS AGAINST FELINE LEUKEMIA VIRUS BY VACCINATION WITH A CANARYPOX VIRUS RECOMBINANT, ALVAC-FL" JOURNAL OF VIROLOGY, NEW YORK, US, US, Bd. 67, Nr. 4, April 1993 (1993-04), Seiten 2370-2375, XP000917684 ISSN: 0022-538X
- KENSIL C R ET AL: "DEVELOPMENT OF A GENETICALLY ENGINEERED VACCINE AGAINST FELINE LEUKEMIA VIRUS INFECTION" JOURNAL OF THE AMERICAN VETERINARY MEDICAL ASSOCIATION, XX, XX, Bd. 199, Nr. 10, 15. November 1991 (1991-11-15), Seiten 1423-1427, XP000616959 ISSN: 0003-1488
- ROBINSON H I: "NEW HOPE FOR AN AIDS VACCINE" NATURE REVIEWS. IMMUNOLOGY, XX, XX, Bd. 2, Nr. 4, April 2002 (2002-04), Seiten 239-250, XP009026179 ISSN: 1474-1733
- ZUR MEGEDE JAN ET AL: "Increased expression and immunogenicity of sequence-modified human immunodeficiency virus type 1 Gag gene" JOURNAL OF VIROLOGY, Bd. 74, Nr. 6, März 2000 (2000-03), Seiten 2628-2635, XP002273330 ISSN: 0022-538X
- HUANG YUE ET AL: "Human immunodeficiency virus type 1-specific immunity after genetic immunization is enhanced by modification of Gag and Pol expression" JOURNAL OF VIROLOGY, THE AMERICAN SOCIETY FOR MICROBIOLOGY, US, Bd. 75, Nr. 10, Mai 2001 (2001-05), Seiten 4947-4951, XP002237413 ISSN: 0022-538X
- FLYNN J N ET AL: "Feline leukaemia virus: Protective immunity is mediated by virus-specific cytotoxic T lymphocytes" IMMUNOLOGY, Bd. 101, Nr. 1, September 2000 (2000-09), Seiten 120-125, XP002273331 ISSN: 0019-2805 in der Anmeldung erwähnt

## Beschreibung

Die Erfindung betrifft einen Impfstoff auf DNA-Basis, durch den Katzen gegen Infektionen mit dem felinen Leukosevirus geschützt werden können.

Das feline Leukosevirus (FeLV) ist ein katzenspezifisches, weltweit verbreitetes Virus, das Auslöser schwerer Erkrankungen ist, und zu den Haupttodesursachen der felinen Population zählt. Derzeit besteht eine Infektionsrate von 12% bis 16% der Katzen sowohl in Europa als auch in den USA.

Ein Teil der Katzen kann die Infektion überwinden; es ist im Gegensatz dazu jedoch auch eine lebenslange Viruspersistenz im Organismus möglich. Latent infizierte Katzen gelten dann als Erregerreservoir.

Eine wirksame Therapie von FeLV-Infektionen, die zu einer Heilung führt, ist zur Zeit nicht möglich. Bestenfalls gelingt ein Zurückdrängen der Krankheit für eine gewisse Zeit. Bestimmte Chemotherapeutika sind auch bei der Katze anwendbar, die Nebenwirkungen sind jedoch wie in der Humanmedizin hochproblematisch. Noch im Versuchsstadium befindet sich die Behandlung mit Interferonen.

Virostatika sind nicht in der Lage, das Virus zu inaktivieren und führen damit ebenfalls zu keinem Heilerfolg.

Eine wirksame Bekämpfung von FeLV-Infektionen kann nur vorbeugend durch Impfung erreicht werden.

### Stand der Technik

Derzeitig erhältliche Impfstoffe gegen FeLV beruhen entweder auf inaktivierten FeL-Viren, auf rekombinant hergestellten Proteinen, sogenannten Subunit-Vakzinen, oder auf dem Einsatz von genetisch modifizierten Lebendvakzinen. Allerdings weisen diese Arten von Impfstoffen neben unzulänglichem Impferfolg eine Reihe weiterer Nachteile auf.

So führen Zubereitungen aus inaktivierten Viren nur bei einem Teil der geimpften Tiere zur erwünschten Immunität. Bei diesen Impfstoffen handelt es sich immer um Proteingemische, in denen hochimmunogene Antigene mit einer großen Menge anderer Proteine um die Präsentation durch das Immunsystem "konkurrieren" müssen. Zudem können nach erfolgter Impfung starke Nebenwirkungen, wie allergische Reaktionen und Autoimmun-Erkrankungen, auftreten.

Ein rekombinanter Impfstoff, der aus biotechnologisch hergestelltem Hüllprotein des FeLV besteht und mit Aluminiumhydroxid und Saponin adjuvantiert ist, ist derzeit ein häufig eingesetzter Impfstoff. Impfungen mit dieser Vakzine führen bei 80 bis 95% der Katzen zum Schutz gegen Leukose (Lutz et al., 1991, J Am Vet Med Assoc; 199(10): 1446-52).

Problematisch ist jedoch das Risiko des Auftretens von Fibrosarkomen an der Impfstelle. Ein weiterer Nachteil dieses Impfstoffes ist, dass die erwirkte Immunität hauptsächlich auf der Bildung virusneutralisierender Antikörper beruht. Neuere Untersuchungsergebnisse (Flynn et al., 2000, Immunology 101, 120-125) zeigen jedoch, dass zur Ausbildung einer schützenden Immunität die zelluläre Immunantwort ebenfalls von sehr großer Bedeutung ist.

Der Einsatz von Lebendvakzinen hat sich zwar hinsichtlich der erzielten Immunität als effektiv erwiesen, birgt jedoch die ständige Gefahr in sich, dass sich die verwendeten Virusstämme durch Mutation oder Rekombination zu neuen pathogenen Virusstämmen entwickeln. Ebenso muss beachtet werden, dass bei der Anwendung von solchen Impfstoffen, die sämtliche Virusstrukturen enthalten, sich auf Grundlage des Antikörperstatus nicht mehr unterscheiden lässt, ob die Tiere infiziert oder immunisiert sind. Aus diesen beiden Gründen eignen sich diese Impfstoffe nicht für die Praxis.

Ein weiteres Beispiel für eine aus infektions- oder replikationsfähigen Viren bestehende Vakzine ist ein rekombinantes, FeLV-Oberflächenproteine exprimierendes Kanarienpockenvirus. In Testinfektionen konnten 83% der Tiere vor der Infektion geschützt werden (Jarrett et al., 1993, J of Virology: 2370-2375). Allerdings weist dieser Impfstoff die Nachteile einer Lebendvakzine in bezug auf unvorhersagbare Rekombinationen auf, weiterhin ist er relativ aufwendig und damit teuer herzustellen und zu charakterisieren.

Neben solchen klassischen und modernen rekombinanten Impfstoffen gibt es die Möglichkeit der Impfung mit DNA-Expressionskonstrukten. Dabei wird nur die Information für bestimmte immunogene Teile des Erregers in Form von DNA dem Impfling verabreicht. Nach Impfung werden die FeLV-Antigene von den Zellen der geimpften Katze exprimiert, und stimulieren so eine Immunantwort gegen das Virus.

Diese Möglichkeit, durch Injektion von Antigen kodierenden DNA-Expressionskonstrukten eine Immunantwort gegen dieses Antigen zu erzielen, ist zuerst von Tang und Ulmer für die Maus publiziert worden (Tang et al., 1992, Nature 365, 152-154; Ulmer et al., 1993 Science 259, 1745-1749) und seither in einer großen Menge von Spezies gezeigt worden. Es ist anzunehmen, dass das generelle Prinzip der Impfung mit Immunogen-kodierenden Nukleinsäuren auf alle höheren Tiere übertragbar ist. Hinsichtlich der Wahl geeigneter Antigene, deren Kodierung in Nukleinsäuresequenzen sowie der Wahl des geeigneten lmpfregimes stellen sich dem Fachmann für jede Anwendung eine Reihe zum Teil nur schwer überwindbarer Probleme, was dazu geführt hat, dass bisher noch keine Vakzine auf DNA-Basis in eine Erprobung in der klinischen Phase 2 oder 3 aufgenommen wurde.

Die Impfung von Katzen mit Expressionskonstrukten zur Expression der Gene "env" und "gag" ist in der französischen Patentschrift FR 2 751 223 beschrieben. Die dort skizzierte Erfindung ist allerdings rein hypothetisch und nicht ausreichend offenbart; so sind keinerlei Expressions- und Immunisierungsversuche oder deren Ergebnisse gezeigt. Es handelt sich um eine rein spekulative Anmeldung.

Die US 6, 348, 196 offenbart Plasmide zur Expression des env- und / oder gag-Proteins des FeLV. Derartige Plasmide lassen jedoch nur eine sehr geringe Expressionsrate zu- Aufgrund der geringen Expression, ist mit den Plasmiden gemäß US 6,348,196 eine Immunisierung überhaupt nicht oder nur sehr schwer möglich.

TARTAGLIA (Journal of virology, 1993) offenbart ein rekombinantes Biokonstrukt zur Expression von env- und gag-Genen. Es ist bedingt möglich, mit derartigen Konstrukten eine Immunisierung auf niedrigem Niveau zu erreichen.

KENSIL et al (1991) offenbart ebenfalls ein pharmazeutisches Mittel zum impfen, welches rekombinante Proteine umfasst. Mit den bei KENSIL et al offenbarten pharmazeutischen Mitteln ist keine ausreichende Immunisierung von Tieren möglich.

Auch die US 6,348,196 offenbart ein Mittel zur Immunisierung von Katzen, welches jedoch keine sichere Immunisierung der Tiere ermöglicht.

ROBINSON (2002) offenbart verschiedenen Technologien und Vektoren für Aidsimpfstoffe, die insbesondere das gag-Protein umfassen.

In der WP 00/15824 werden Transfermethoden für spezifische Proteinsäuren offenbart. Diese Druckschrift offenbart auch ein peptidgekoppelten Gentransfer im Zusammenhang mit einer DNA-Immunisierung.

In dem oben genannten Reviews von ROBINSON wird auf die Publikationen von MEGEDE et al (2000) und HUANG et al (2001) verwiesen. Diese Publikationen offenbaren eine Kodonoptimierung bei viralen Genen zu deren Verwendung in DNA-Impfstoffen.
Trotz der unterschiedlichen Herangehensweise und der unterschiedlichen Strategien der oben genannten Offenbarungen haben alle den selben technischen Nachteil: mit ihnen ist keine zufriedenstellende Immunisierung des Zielorganismus, insbesondere von Katzen, möglich.

Versuche zur DNA-Immunisierung auf dem Gebiet des FeLV, die allerdings nicht zu einem überzeugenden Erfolg führten, sind bekannt (Jarrett et al., 2000 Immunology 101, 120-125). In der vorzitierten Arbeit war das Gesamtgenom mit einer Polymerasedeletion als Expressionskonstrukt inokuliert worden. Der klinische Erfolg der Impfung schlug sich allerdings nicht in einem Schutz der Katzen vor Infektion und Virämie nieder. Neben diesem praktischen Nachteil des zitierten Impfversuchs hat die Verwendung von Deletionsmutanten oder deren Genom zur Impfung den Nachteil, dass die Gefahr besteht, dass aus einem deletierten Virus durch Rekombination mit endogenen oder exogenen Virussequenzen infektiöse, neuartige Pathogene entstehen.

Im Gegensatz zu der zitierten Arbeit von Jarrett war das Ziel der Bemühungen der vorliegenden Erfindung, nur isolierte Antigene des FeLV zur Expression zu bringen. Vorversuche zeigten allerdings, dass durch Inokulation von Expressionskonstrukten, die homologe Wildtyp-Sequenzen des "env"- und "gag"-Gens des FeLV unter Kontrolle des Cytomegalievirus (CMV) early-immediate Promotors kodierten, keine Antikörperproduktion in Katzen provozierbar war. Weitere Versuche zeigten ebenfalls, dass die betreffenden Sequenzen in menschlichen und aus Katzen gewonnenen Zelllinien nicht oder nur in sehr geringem Maße exprimiert wurden. Dieses Phänomen ist für Sequenzen des HI-Virus sowie andere Lentiviren bekannt (Wagner et al., 2000, Hum Gene Ther 11 (17), 2403-2413). Die Expression der Wildtypsequenzen in der infizierten Zelle ist dabei abhängig von der vorhergehenden Expression des viral kodierten Proteins "rev".

Die Expressionskontrolle des nicht zur Klasse-der-Lentiviren gehörenden Retrovirus FeLV ist unbekannt und ein der "rev"-Kontrolle ähnlicher Mechanismus ist weder gezeigt noch in der Literatur postuliert worden.

Ebenfalls bekannt ist, dass durch Optimierung der Kodonbenutzung (Codon Usage Optimization) innerhalb des Expressionskonstruktes an die präferentiell in Säugern benutzten Kodons die Expression von Proteinen erheblich gesteigert werden kann (Grantham et al., Nucleic Acids Res 1980, 9:1893-912). Diese Methode wurde bereits erfolgreich zur Expressionssteigerung verschiedener viraler Strukturproteine des HIV-1 und SIV angewandt. Der Effekt beruht auf der Umgehung der "rev"-abhängigen Transportwege für das extrem AT-reiche Transkript dieser späten Proteine im Replikationszyklus der Lentiviren. So gelang es durch Kodonoptimierung der DNA Sequenzen des "env" und des "gag" Proteins des menschlichen HI-Virus, weitaus höhere Antikörpertiter gegen diese synthetischen Antigene in Mäusen zu erzielen, als es durch die Wildtypsequenzen möglich war (Haas et al., 1998, J Virol. 72: 1497-503, Wagner et al., Hum Gene Ther. 2000,17:2403-13). Die Herstellung und der Gebrauch solcherart optimierter Sequenzen zur Vakzinierung gegen HIV-1 ist auch bekannt aus der WO 00/029561 und der WO 97/48370.

Ein anderes Problem besteht in der Applikation der die immunogenen Antigene oder Teile davon kodierenden DNA. Ein Nachteil der im Moment zum DNA Transport (Transfektion) verwendeten Vektoren besteht darin, dass entweder Vektoren viralen Ursprungs eingesetzt werden, die vom Aspekt der Sicherheit Probleme aufwerfen (Lehrman, 1999, Nature 401: 517-518), oder aber Plasmide genutzt werden. Da Plasmide durch bakterielle Fermentation gewonnen werden, enthalten sie neben dem gewünschten Gen auch die für ihre Vervielfältigung und Selektion notwendige DNA sowie üblicherweise Resistenzgene gegen bei der Fermentation verwendete Antibiotika. Die Problematik wird in der WO 98/21322 ausführlich diskutiert. Erwähnt sei, dass bei der Verwendung von Genexpressionskonstrukten auf Basis von Plasmid-DNA das inhärente Risiko der Verbreitung von Antibiotikaresistenzgenen, welches insbesondere bei großangelegten Schutzimpfungen nicht vertretbar ist, besteht.

Eine andere Art von DNA-Vektor sind kovalent geschlossene minimalistische DNA-Konstrukte, wie sie in der EP 0 914 318 B1 offenbart werden. Insbesondere deren Anwendung in Form von peptid-gekoppelten DNA-Konstrukten führt zu einer überraschenden, qualitativ verbesserten Immunantwort im Vergleich zu unmodifizierter Plasmid-DNA (siehe auch DE 101 56 679.4 und DE 101 56 678.6).

Neben den Nachteilen, die durch die bisherigen Gentransfermethoden bedingt sind, ist es bisher nicht gelungen, einen effektiven und sicheren Impfstoff gegen FeLV zu entwickeln. Bis heute beschränkt sich die Behandlung einer FeLV-Infektion auf die Stärkung der Abwehrkräfte der Tiere und die Behandlung der Begleit- und Sekundärinfektionen. Die vorhandenen Impfstoffe bringen die eingangs genannten Nebenwirkungen mit sich.

Ausgehend von diesem Stand der Technik ist es Aufgabe der vorliegenden Erfindung, einen Impfstoff zur Verfügung zu stellen, der in Katzen zu einem Schutz vor Infektionen mit FeLV führt, sowie geeignete diagnostische Werkzeuge zur Verfügung zu stellen.

### Lösung der Aufgabe und Vorteile der Erfindung

Erfindungsgemäß wird dieses Ziel dadurch erreicht, dass Katzen mit einem Gemisch (Cocktail) aus synthetisch hergestellten, kodon- und spleiß-Signal-optimierten DNA-Sequenzen immunisiert werden, die für Strukturproteine ("gag"), sowie das wichtigste Membranprotein ("env") des FeLV gemäß Seq. ID6 und Seq. ID9 oder gemäß Seq. ID6 and Seq. ID10 kodieren.

Im Zuge der Kodon-und Spleiß-Optimierung der verwendeten DNA-Sequenzen wurden auch mutagenisierte DNA-Sequenzen erhalten, welche zur Substitution einzelner Aminosäuren bei den Strukturproteinen ("gag") und dem wichtigsten Membranprotein ("env") des FeLV führten. Überrachenderweise wiesen diese Proteine mit veränderter Aminosäuresequenz die erfindungsgemäßen Vorteile auf. Daher ist im Sinne dieser Erfindung die Kodon- und Spleiß-Optimierung eine Strategie zur Veränderung der Aminosäuresequenz der Strukturproteine ("gag"), sowie des wichtigsten Membranproteins ("env") des FeLV.

### Im Sinne der Erfindung bedeuten hierbei

- "env":: Gensequenz, die für die viralen Hüllproteine der inneren Virusverpackung des felinen Leukosevirus kodiert
- "gag":: Gensequenz, die für die viralen Strukturproteine der inneren Virusverpackung des felinen Leukosevirus kodiert
- FeLV:: feline Leukosevirus
- WT:: Wildtyp
- WT "env":: Wildtyp der "env" DNA Sequenz, entnommen der NCBI-Datenbank, Acc. No. M12500
- WT "gag":: Wildtyp der "gag" DNA Sequenz, gewonnen aus dem Blut infizierter-Katzen (siehe Beispiel 1), keine Sequenz aus einer Datenbank, jedoch homolog zu einer solchen
- NLS:: Kern-Lokalisierungssequenz (Nuclear Localization Signal)
- ODN:: Oligodesoxynukleotid
- PCR:: Polymerase Chain Reaction
- LeadFeLVenv (FeLVenv):: FeLV "env" Sequenz mit Signalsequenz
- LeadFeLVenvgp85 (FeLVenvgp85):: FeLV "env" Sequenz (gp85) mit Signalsequenz

Die Gensequenz "gag" kodiert für die viralen Strukturproteine der inneren Virusverpackung, die Gensequenz "env" für die viralen Hüllproteine. Das Protein, das die höchste Immunogenität der in der "env" Sequenz kodierten Proteine besitzt, ist das Glykoprotein gp70. Gegen das gp70 werden im Katzenorganismus virusneutralisierende Antikörper gebildet. Diese Antikörper stellen die erste Immunantwort nach dem Eindringen des Erregers in den Körper dar, die unter Umständen schon ausreichend zur Überwindung der Infektion sein kann.

Ob membranständige oder sekretorische Proteine besser geeignet sind, virusneutralisierende Antikörper zu induzieren, wird kontrovers diskutiert. Aus diesem Grund wurden zwei verschiedene für "env" kodierende Konstrukte hergestellt. Da bekannt ist, dass die p15 Sequenz der "env" Gensequenz mindestens einen immunmodulatorisch wirksamen Sequenzabschnitt enthält, der die Antikörperbildung unterdrückt (Haraguchi et al., 1997, Journal of Leukocyte Biology, 61, 654-666), wurde neben einem Konstrukt kodierend für gp70 und p15 (gp85), ein weiteres hergestellt, welches nur das gp70 enthielt und damit zur Expression des sekretorischen "env" Proteins ohne Transmembranteil führt.

Eine Vakzine, die sowohl virusneutralisierende Antikörper gegen das gp70, als auch eine T-Zell-vermittelte Immunantwort induzieren kann, stellt demnach eine deutliche Verbesserung gegenüber den bisher erhältlichen Impfstoffen dar und könnte gegebenenfalls auch zur Therapie infizierter Katzen eingesetzt werden.

Um mehr Antigen in-vivo zu exprimieren und damit eine stärkere Immunantwort auszulösen, die sich in einem wirksamen und dauerhaften Schutz vor der FeLV-Infektion zeigt, wurden die Wildtyp Sequenzen von "gag" und "env" optimiert. Unter Optimierung soll die Kodon-Anpassung, auch als "Codon-Usage-Optimization" bezeichnet, verstanden werden.

Jede Aminosäure kann durch mehrere Kodons verschlüsselt werden. Die Häufigkeit, mit der die einzelnen Kodons während der Transkription abgelesen werden, variiert sehr stark zwischen Viren, Bakterien und Vertebraten. Dementsprechend variiert auch die Häufigkeit der entsprechenden tRNAs in der Zelle. Virale Genome haben zum Teil eine von der Wirtszelle abweichende Kodon-Nutzungsfrequenz, was wahrscheinlich ein Element der Expressionskontrolle der Viren ist. Durch Adaption der Sequenz an das wirtsspezifische Kodon-Nutzungsmuster können diese viralen Steuermechanismen unterlaufen sowie die Expression von Antigen erheblich gesteigert werden.

Deshalb war das Ziel der Experimente, durch Umschreibung der viralen Sequenzen in Sequenzen, die eine für Vertebratengenome optimale Kodon-Nutzung repräsentieren, eine viel stärkere Expression der Antigene zu erreichen. Dazu wurde eine Klonierungsstrategie entwickelt, die die Synthese dieser optimierten DNA-Sequenz aus Oligonukleotiden ermöglichte.

Die synthetischen Sequenzen wurden in Plasmide inseriert, in E.coli vervielfältigt, zur Kontrolle sequenziert und anschließend in Zellen einer Katzenzelllinie transfiziert, um die Expression der kodierten Proteine zu testen.

Der Nachweis der Expression der Antigene wurde mit Hilfe von Westemblots geführt.

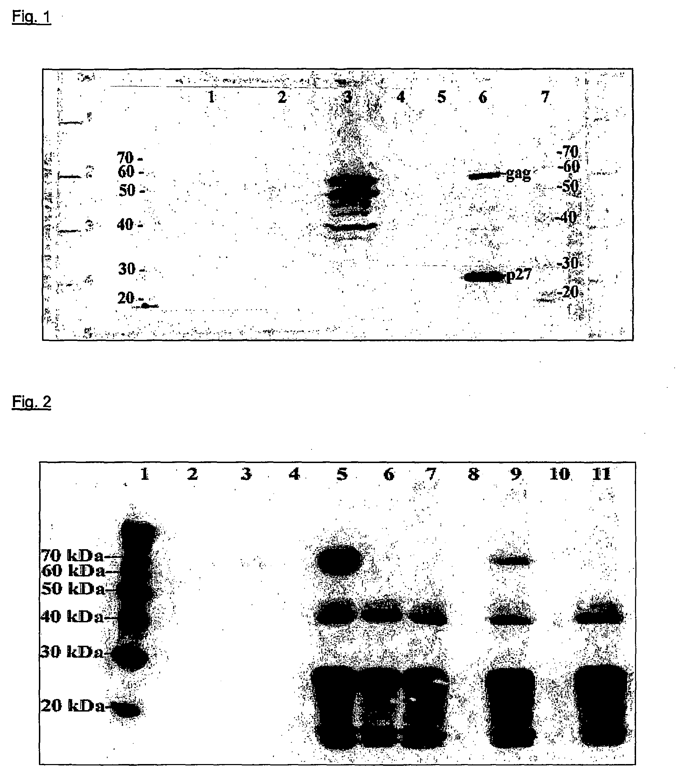

Der Nachweis der Expression der Proteine durch die FeLV-Wildtyp Sequenzen (WT) von "env" und "gag" erfolgte mit Hilfe des Westernblots. Durch Immunpräzipitation konnte "env" ebenso wie "gag" nur durch sehr schwache Banden nachgewiesen werden. Überraschenderweise war dies auch für die kodon-optimierten "env"-Sequenzen der Fall. Nur bei "gag" führte die Kodon-Optimierung zu einer deutlich verbesserten Expression, wie Fig. 1 belegt. Nach Überprüfung zahlreicher anderer Hypothesen, die die mangelhafte Expression der synthetischen Gene erklären sollten, wurde die synthetische "env" Sequenz auf durch bioinformatorisch vorhergesagte Spleißsignale (splice-sites) überprüft. Eine Reihe der vom verwendeten Programm (Complign PPC, Mac Molly Tetra Version 3.10a (Softgene GmbH)) vorhergesagten Spleißsignale wurde durch Punktmutation beseitigt, um die Hypothese zu verifizieren, dass das Auftreten solcher Strukturen die Expression der Gene im Zusammenhang mit dem verwendeten Promoter behindere. Es gelang überraschenderweise durch diese Maßnahme, "env" im Westernblot als starke Proteinbande nachzuweisen (s. Fig. 2). Die erfindungsgemäßen synthetischen Antigensequenzen zeigen durch die kräftigen Banden die erfolgreiche und verstärkte Expression der Antigene.

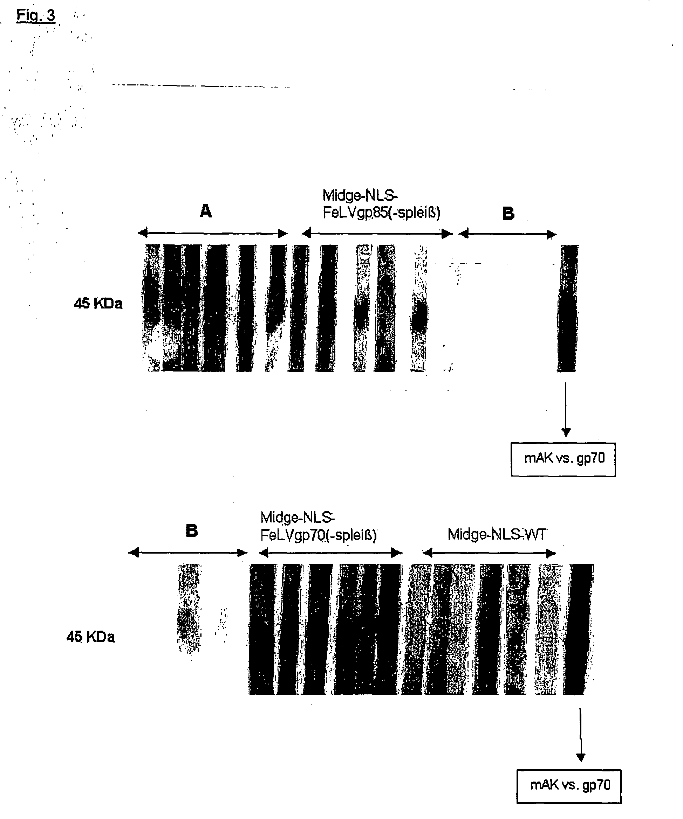

Es wird generell ein Zusammenhang zwischen der Expressionsstärke eines Expressionssystems und der resultierenden Stärke der Immunantwort vermutet, wenn auch zahlreiche Befunde nahe legen, dass weder ein linearer Zusammenhang besteht, noch zwingend jede Behandlung mit Expressionsvektoren Immunität in der gewünschten Stärke nach sich zieht (Wherry et al., Journal of Immunology 2002, 168 pp 4455-4461). Deshalb wurden nach Vorliegen der *In-vitro*-Expressionsergebnisse Mäuse mit peptid-gekoppelten Expressionskonstrukten immunisiert, die für das kodonoptimierte und spleißsignal-optimierte "env" kodierten. Die Vorteile solcher peptidgekoppelten Konstrukte bei der Herbeiführung einer Immunantwort sind in den Schriften EP 0 941 318 B1 und DE 101 56 678 A1 ausführlich erläutert. Zur Klärung der immunologischen Bedeutung des p15 Proteins des "env" bei der Auslösung einer Immunantwort wurden beide erfindungsgemäße für "env" kodierende Sequenzen eingesetzt (Seq.ID 7, 8, sowie 9 und 10). Die Seren der immunisierten Mäuse wurde auf spezifische Antikörper gegen das FeL-Virusprotein "env" mittels Westernblot untersucht. Anhand der Antikörperlevel nach der Zweitimmunisierung in Woche 4 ist erkennbar, dass die synthetischen Konstrukte auch *in-vivo* zu einer starken Stimulation der Antikörperbildung führen. Im Vergleich zeigten fünf von sechs Mäuse der Gruppe 4 eine starke Immunantwort auf die erfindungsgemäße Antigensequenz, wohingegen der WT (Gruppe 1) zum Auslösen einer schwachen Antikörperantwort bei nur zwei von sechs Tieren führte (s. Fig. 3).

Als DNA-Expressionskonstrukte können Plasmide eingesetzt werden, bevorzugt werden aber erfindungsgemäß minimalistische immunologisch definierte Genexpressionskonstrukte verwendet. Dabei handelt es sich um lineare, kovalent geschlossene Expressionskassetten, die lediglich aus dem CMV Promotor, einem Intron, der entsprechenden Gensequenz und einer Polyadenylierungssequenz bestehen. Diese kovalent geschlossenen minimalistische DNA-Konstrukte werden im folgenden als Midge Vektoren bezeichnet (MIDGE: MINIMALISTIC IMMUNOLOGI-CALLY DEFINED GENE EXPRESSION VECTOR); vgl. EP 0 941 318 B1. Die Midge-Konstrukte haben den Vorteil, dass mit ihnen auf Strukturen verzichtet werden kann, die für deren medizinische Wirkung nicht essentiell sind, was die Nachteile der herkömmlichen Genfähren vermeidet.

Zur Transfektion können biologische, chemische und/oder physikalische Methoden eingesetzt werden, die zum Stand der Technik gehören, beispielsweise Transfektion mittels ballistischem Transfer. In einer bevorzugten Ausführungsform der Erfindung erfolgt die Transfektion mittels intradermaler Injektion durch Spritzen oder nadelfreier Injektionsgeräte.

Die Erfindung hat Expressionskonstrukte zum Gegenstand, die zur Expression von Antigenen des FeLV in Säugerzellen führen. Die Erfindung stellt somit einen Impf stoff zur Verfügung zu stellen, der in Katzen zu einem Schutz vor Infektionen mit FeLV führt und den Sicherheitsaspekt berücksichtigt. Erfindungsgemäß wird auf herkömmliche Adjuvantien verzichtet, so dass damit die Gefahr der Fibrosarkombildung an der Injektionsstelle ausgeschlossen werden kann.

Weitere vorteilhafte Methoden sind die biologischen Transfektionsmethoden wie der peptidgekoppelte Gentransfer. Hierbei beispielsweise wird ein DNA-Expressionkonstrukt, das für mindestens die erfindungsgemäße "env" und mindestens die erfindungsgemäße "gag" Sequenz des FeLV kodiert, kovalent mit einem Oligopeptid verbunden, welches vorzugsweise die Kem-Lokalisierungssequenz (Nuclear Localization Signal = NLS) aus dem Simian Virus SV-40 ist.

Nach den positiven Ergebnissen im Mausexperiment wurden Katzen mit den Expressionskonstrukten immunisiert und auf ihren Antikörperstatus untersucht.

Das beigefügte Sequenzprotokoll, welches Bestandteil der Anmeldung und vorliegenden Beschreibung ist, gibt folgende Sequenzen wieder:

| Seq.ID | Sequenzname/-beschreibung |
|---|---|
| Seq.ID1 | DNA-Sequenz des Wildtyps des "env"-Gens |
| Seq.ID2 | DNA-Sequenz des Wildtyps des "gag"-Gens |
| Seq.ID3 | Proteinsequenz des Wildtyps des "env"-Gens |
| Seq.ID4 | Proteinsequenz des Wildtyps des "gag"-Gens |
| Seq.ID5 | DNA-Sequenz des mutagenisierten "gag"-Gens |
| Seq.ID6 | Proteinsequenz des mutagenisierten "gag"-Gens |
| Seq.ID7 | DNA-Sequenz des mutagenisierten "env"-Gens (gp85). Die gp70 Sequenz ist hierbei um die für das immunogene Protein p15 kodierende Nukleotidsequenz verlängert. |
| Seq.ID8 | DNA-Sequenz des mutagenisierten "env"-Gens (gp70) |
| Seq.ID9 | Proteinsequenz des mutagenisierten "env"-Gens (gp85) |
| Seq.ID10 | Proteinsequenz des mutagenisierten "env"-Gens (gp70) |
| Seq.ID11 | DNA-Sequenz des Wildtyps des "env"-Gens (gp70), entnommen der Seq.ID1 (NCBI-Datenbank, Acc. No. M12500). |
| Seq.ID12 bis Seq.ID40 | Sequenzen der verwendeten Primer entsprechend den nachfolgenden Beispielen. |

Es ist beschrieben ein DNA-Expressionskonstrukt zur Expression von Genprodukten des Felinen Leukosevirus (FeLV) in Katzenzellen, bestehend aus einer in Feliden operablen Promotorsequenz und mindestens einer Nukleotidsequenz, die verwandt ist mit einer für ein originäres Strukturprotein ("gag") und/oder Membranprotein ("env") des FeLV kodierenden Wildtyp-Nukleotidsequenz, wobei besagte Nukleotidsequenz des FeLV verändert ist und keine offenen oder versteckten Splice-Donor und/oder Akzeptorsequenzen aufweist und für ein mit dem originären Strukturprotein ("gag") und/oder dem originären Membranprotein ("env") des FeLV hochgradig homologes aber nicht identisches Protein oder einen hochgradig homologen aber nicht identischen Teil derselben kodiert. Die mit dem originären Strukturprotein ("gag") und/oder dem originären Membranprotein ("env") des FeLV hochgradig homologen aber nicht identischen Proteine weisen eine Homologie zum korrespondierenden Wildtyp von wenigstens 98% auf. Bevorzugt ist ein Expressionskonstrukt enthaltend die Sequenzen Seq.ID5, Seq.ID7 und/oder Seq.ID8.

Die Struktur- und/oder Membranproteine werden ganz oder teilweise durch die entsprechenden Nukleotidsequenzen kodiert. Bei dem Expressionskonstrukt handelt es sich entweder um ein Plasmid oder um ein solches Konstrukt, bei dem die immunisierenden Polynukleotidsequenzen als Expressionskonstrukte vorliegen, die aus kovalent geschlossenen linearen Desoxyribonukleinsäuremolekülen bestehen, welche einen linearen Doppelstrangbereich aufweisen und wobei die doppelstrangbildenden Einzelstränge durch kurze einzelsträngige Schlaufen aus Desoxyribonukleinsäurenukleotiden verknüpft sind, und wobei die doppelstrangbildenden Einzelstränge nur aus der kodierenden Sequenz unter Kontrolle eines im zu impfenden Tier operablen Promotors und einer Terminatorsequenz besteht.

Zur besseren Transfektion kann das Expressionskonstrukt mit einem oder mehreren Peptiden kovalent verknüpft sein. Bevorzugt ist ein Peptid aus 3 bis 30 Aminosäuren, von denen mindestens die Hälfte basische Aminosäuren aus der Gruppe Arginin und Lysin kommen, insbesondere ein Peptid mit der Aminosäuresequenz PKKKRKV (Polin-Lysin-Lysin-Lysin-Arginin-Lysin-Valin).

Erfindungsgemäß sind aber auch Proteine vorgesehen, welche ein mit dem originären Strukturprotein ("gag") des Felinen Leukosevirus (FeLV) hochgradig homologes Protein (Seq.ID6) oder mit dem originären Membranprotein gp85 ("env") des FeLV (Seq.ID9) oder mit dem originären Membranprotein gp70 ("env") des FeLV (Seq.ID10) darstellen. Diese Proteine wiederum können zur Antikörperproduktion (monoklonale oder polyklonale Antikörper) verwendet werden, die wiederum Bestandteil von diagnostischen Kits zur Diagnose von Infektionen bei Katzen mit dem Felinen Leukosevirus sind.

Das erfindungsgemäße Expressionskonstrukt ist als Bestandteil eines Arzneimittels, insbesondere einem Impfstoff zur Erzeugung einer präventiven und/oder therapeutischen Immunität bei Feliden, insbesondere der Katze, vorgesehen.

Weitere vorteilhafte Ausgestaltungsformen der Erfindung ergeben sich aus den Unteransprüchen und der Beschreibung. Die überraschende Wirkung des erfindungsgemäßen Arzneimittels (als Impfstoff zur FeLV Therapie), sowie das erfindungsgemäße Verfahren wird anhand der Figuren und Ausführungsbeispiele deutlich. Dabei bedeuten:
- Midge-NLS-FeLVenvgp85(-spleiß) oder Midge-NLS-FeLVgp85(-spleiß): NLS-gekoppelter Midgevektor kodierend für die kodon-u. spleißsignal.optimierte "env"-Sequenz mit p15 (gp85)
- Midge-NLS-FeLVenvgp70(-spleiß) oder Midge-NLS-FeLVgp70(-spleiß): NLS-gekoppelter Midgevektor kodierend für die kodon-u. spleißsignal.optimierte "env"-Sequenz ohne p15 (gp70)
- Midge-NLS-WT: NLS-gekoppelter Midgevektor kodierend für den WT des "env"-Gens
- mAK vs. gp70: monoklonaler Antikörper gegen gp70
- Positivkontrolle: Impfstoff Leucogen
- Puffer: PBS, Negativkontrolle

Es zeigt:
- Fig. 1:: in-vitro Expressionsvergleich des WT "gag"-Proteins und des kodon optimierten "gag"-Proteins. Aufgetragen sind Katzenzelllysate, die zuvor mit folgenden Konstrukten transfiziert wurden. Das "gag" Vorläufer-Protein hat eine Größe von 55 kDa:
Spur 1 u. 2: für "gag"-WT kodierende Expressionsvektoren
Spur 3: für kodon-optimiertes "gag" kodierende Expressionsvektoren
Spur 4: nichtinfizierte Katzenzellen, Negativkontrolle
Spur 5: leer
Spur 6: mit Virus infizierte Katzenzellen, Positivkontrolle
Spur 7: Boa-Proteinmarker
Die Expression durch den WT führt zu sehr schwachen Proteinbanden (1 und 2), wohingegen eine starke Expression durch die erfindungsgemäße Sequenz erreicht wird (3). Zum Vergleich dienen infizierte Katzenzellen (6). Das "gag" bezeichnet ein Vorläuferprotein, welches in der infizierten Zelle mit Hilfe von Proteasen in Strukturproteine des FeLV zerlegt wird. Das am stärksten immunogen wirkende Strukturprotein ist das p27, das deshalb von einem Antiserum gegen das Gesamtvirus sehr gut erkannt wird. Dadurch ist es erklärlich, das in Spur 6 sowohl eine 55 kDa für das gesamte "gag" als auch eine 27kDa Bande erkennbar ist. Im Gegensatz dazu enthält Spur 3 keine Viruspartikel, sondern mit dem "gag" Gen transfizierte Zellen, dies führt offensichtlich nicht zum Abbau des "gag" Genes in die Virusproteine mit Hilfe von Proteasen, sondern zur unspezifischen Degradation des gesamten Vorläuferproteins durch zelleigene Proteasen.
- Fig.2:: in-vitro Expressionsvergleich des WT "env"-Gens und der kodon-u. spleißsignaloptimierten "env"-Sequenz (gp85). Aufgetragen sind Katzenzelllysate, die zuvor mit folgenden Konstrukten transfiziert wurden:
Spur 1: Boa-Proteinmarker
Spur 2: nichtinfizierte Katzenzellen, Negativkontrolle
Spur 3: mit Virus infizierte Katzenzellen, Lysat, Positivkontrolle
Spur 4: leer
Spur 5: mit Virus infizierte Katzenzellen, Präzipitat, Positivkontrolle
Spur 6: nichtinfizierte Katzenzellen, Präzipitat, Negativkontrolle
Spur 7: weitere Negativkontrolle zum spezifischen "env"-Nachweis
Spur 8: leer
Spur 9: für kodon- und spleißsignaloptimiertes FeLVenvgp85(-spleiß) kodierende Expressionsvektoren
Spur 10: leer
Spur 11: für kodonoptimiertes FeLVenvgp70(-spleiß) kodierende Expressionsvektoren Die Kontrolle in Spur 5 führt zu einer deutlichen Proteinbande, die als Positivkontrolle für die Expression des envgp85 genutzt werden kann. Spur 9 zeigt, das die erfindungsgemäße FeLVenvgp85(-spleiß) Sequenz zur Expression des gp85 Proteins führt. In Spur 11 ist das erfindungsgemäße FeLVenvgp70(-spleiß) aufgetragen. Die Abwesenheit der 70kDa Bande beruht darauf, das das envgp70 im Gegensatz zum gp85 ein sekretorisches Protein ist, das sich im Zeillysat schwer oder kaum nachweisen läßt.
- Fig.3:: in-vivo Ergebnisse nach Immunisierung von Mäusen mit für "env"-kodierenden Expressionskonstrukten. Dabei bedeuten:
A: Positivkontrolle
B: Puffer
Die Antikörperbestimmung erfolgte in Woche 4 nach der Zweitimmunisierung. In Gruppe 3 sind 3 von 6 Mäuseseren antikörperpositiv (Gelspuren unter dem Pfeil: Midge-NLS-FeLVgp85(-spleiß)), in der 4. Gruppe 5 Seren stark positiv und 1 schwach positiv (Gelspuren unter dem Pfeil: Midge-NLS-FeLVgp70(-spleiß)). Dagegen zeigen die mit dem WT immunisierten Tiere der Gruppe 5 nur in zwei Fällen sehr schwache positive Antikörpersignale (Gelspuren unter dem Pfeil: Midge-NLS-WT). Der Versuch zeigt, das die optimierten Sequenzen auch in-vivo zu einer deutlich verstärkten Antikörperbildung gegenüber dem WT führen und bestätigt die Ergebnisse der in-vitro Experimente.

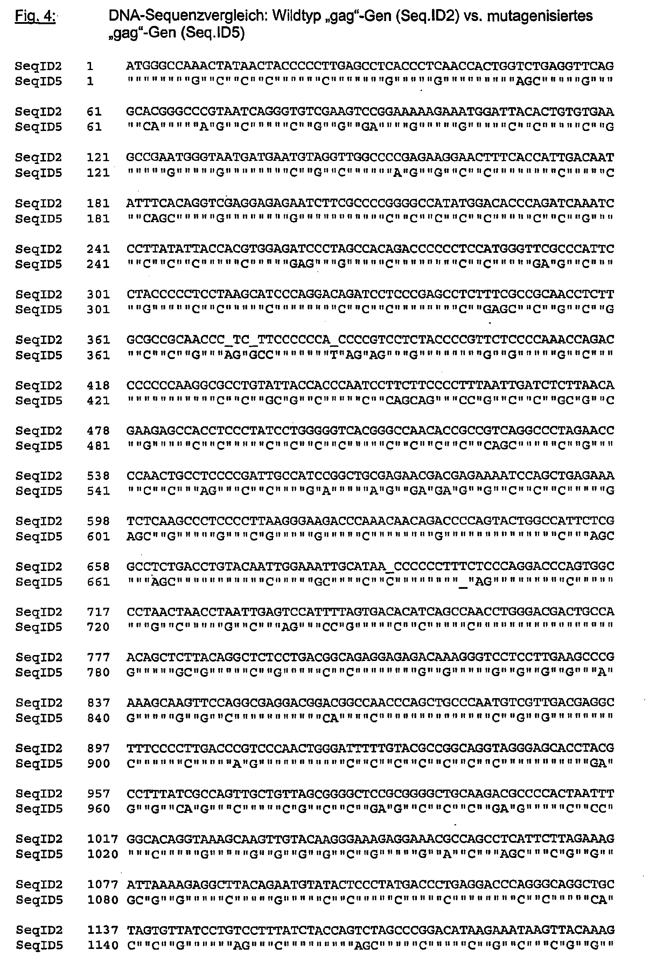

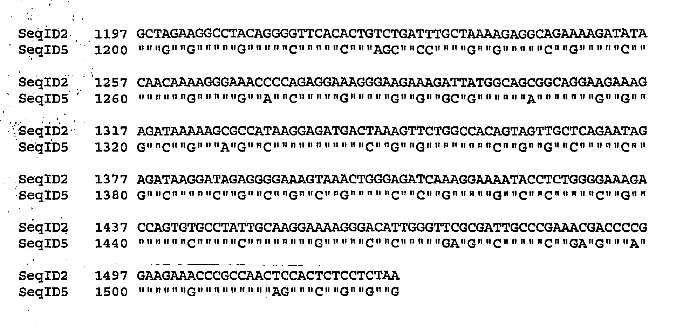

- Fig. 4:: DNA-Sequenzvergleich Wildtyp "gag"-Gen (Seq.lD2) vs. :kodon-optimiertes-"gag"-Gen (Seq. ID5). Ähnlichkeit: 74.51%

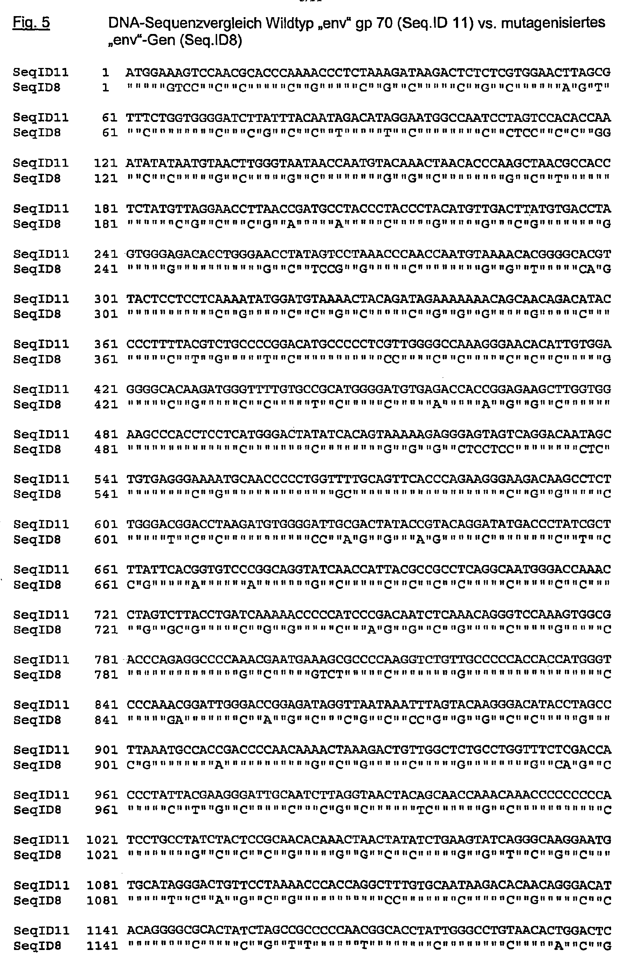

- Fig. 5:: DNA-Sequenzvergleich Wildtyp "env" (gp70-Region aus Seq. ID1) vs. kodon- und spleißsignal optimiertes "env"-Gen (gp70; Seq.lD8). Ähnlichkeit: 75.75%

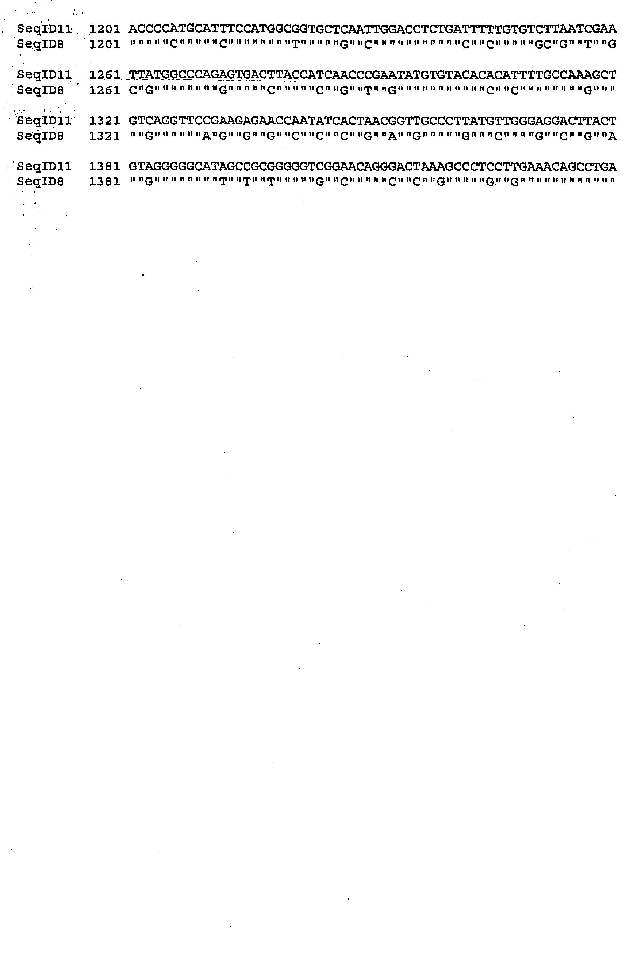

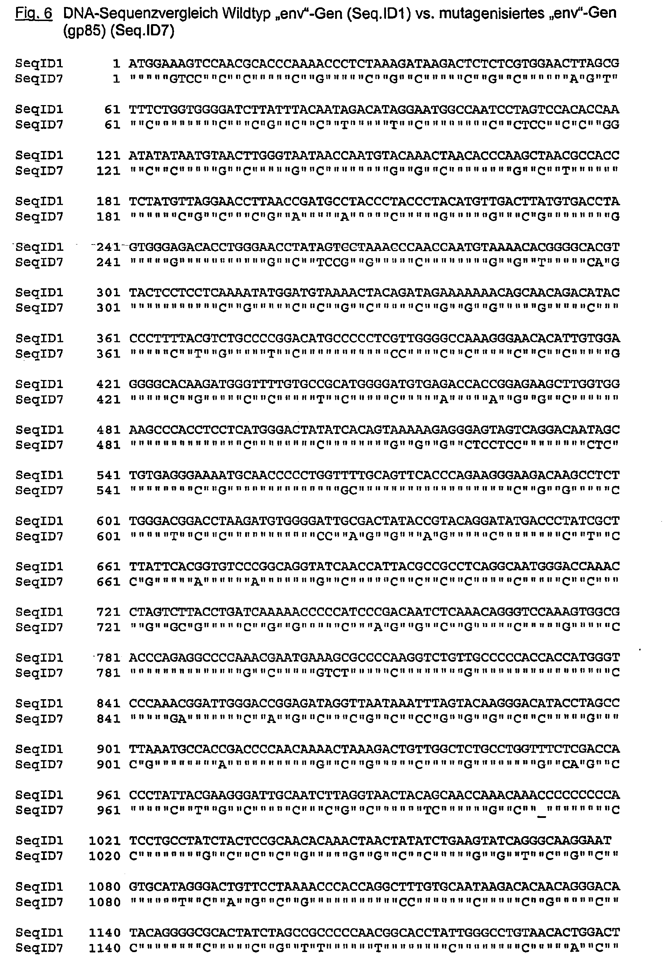

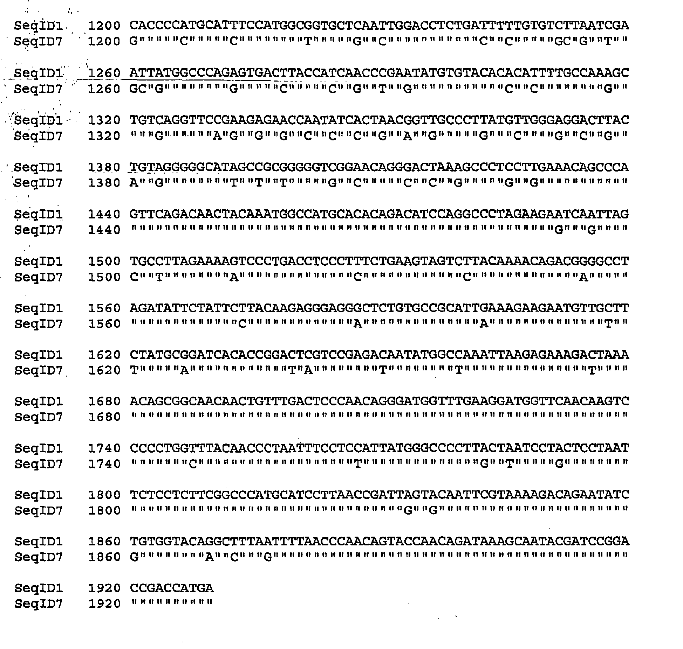

- Fig. 6:: DNA-Sequenzvergleich Wildtyp "env"-Gen (Seq.ID1) vs. kodon- und spleißsignal optimiertes "env"-Gen (gp85) (Seq.lD7). Ähnlichkeit: 80.25%

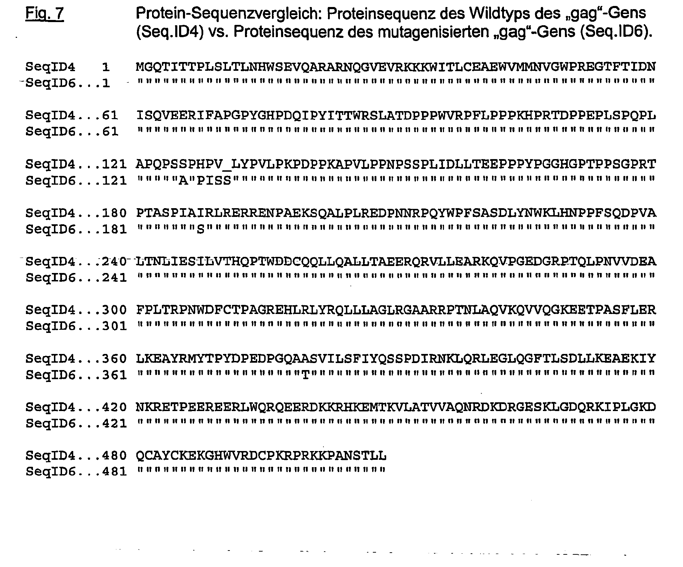

- Fig.7:: Protein-Sequenzvergleich: Proteinsequenz des Wildtyps des "gag"-Gens (Seq.lD4) vs. Proteinsequenz des kodon-optimierten "gag"-Gens (Seq.ID6). Ähnlichkeit : 98.62%

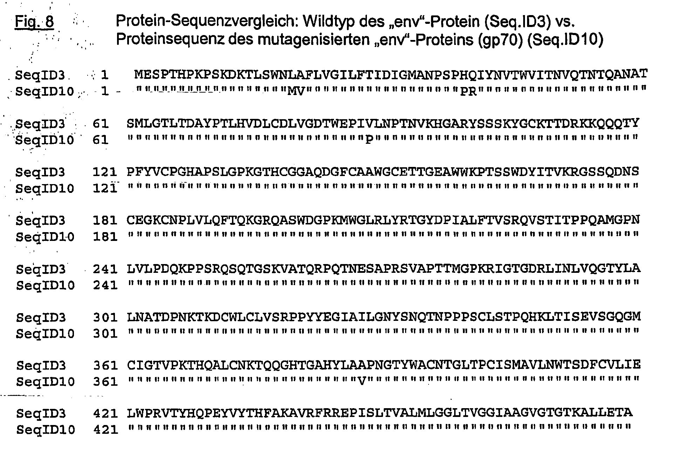

- Fig. 8:: Protein-Sequenzvergleich: Wildtyp des "env"-Protein (Seq.ID3) vs. Proteinsequenz des kodon- und spleißsignal-optimierten "env"-Proteins (gp70) (Seq.ID10). Ähnlichkeit: 98,75%

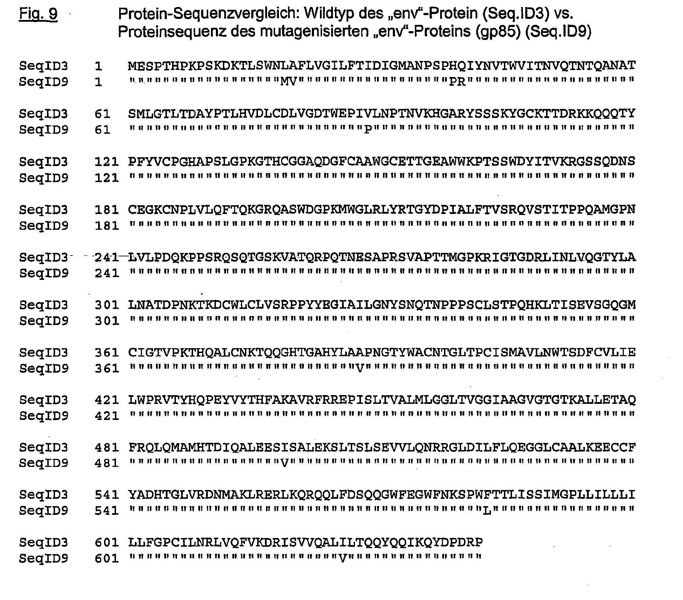

- Fig. 9:: Protein-Sequenzvergleich: Wildtyp des "env"-Protein (Seq.ID3) vs. Proteinsequenz des kodon und spleißsignal-optimierten "env"-Proteins (gp85) (Seq.ID9). Ähnlichkeit : 98.60%

Die Ergebnisse belegen, dass die codon- und spleißsignaloptimierten DNA-Sequenzen sowohl des "env" als auch des "gag" Gens des FeLV eine Homologie zum entsprechenden Wildtyp von wenigstens 74 % aufweisen. Daraus resultierend ergeben sich Proteinsequenzen des "env" und des "gag" Proteins mit einer Homologie zum korrespondierenden Wildtyp von wenigstens 98%. Es sind durchaus andere Optimierungen an der DNA-Sequenz des "env" als auch des "gag" Gens des FeLV denkbar, die zu einem ähnlichen Resultat führen, dass heißt einer hohen Homologie zwischen originärem Wildtyp und der (aus der Optimierung resultierenden) Proteinsequenz. Auch solche Optimierungen werden als im Sinne der Erfindung verstanden.

### Beispiel 1: Wildtyp (WT)-Sequenzen

Die Wildtyp-Sequenzen der ausgewählten Antigene, insbesondere für das "gag" Gen, wurden aus dem Blut infizierter Katzen gewonnen. Die DNA-Sequenz für "env" WT ist in Seq. ID 1 (NCBI-Datenbank, Acc. No. : M12500), für "gag" WT in Seq. ID 2 wiedergegeben. Die entsprechenden Aminosäuresequenzen in Seq. ID 3 ("env") und Seq.ID 4 ("gag").

### Primer für "aag" WT:

Zur Beseitigung von zwei Eco 31 |Schnittstellen wurden 3 PCR mit folgenden Mutationsprimern durchgeführt:
gag-mut1-rneu (Seq.ID12):
   AATTAAGAGCTCCACGTCTCCCCCCGCTAACAGCAACTGGCG
gag-mut2-1 (Seq.ID13):
   AATTAAGAGCTCCAGGTCTCCGGGGCTCCGCGGGGCTGCAAGACG
gag-mut3-r (Seq.ID14):
   AATTAAGAGCTCCACGTCTCCTTCCCTTTTGTTGTATATCTTTTCTGC
gag-mut4-1 (Seq.ID15):
   AATTAAGAGCTCCAGGTCTCCGGAAACCCCAGAGGAAAGGGAAGAAAG

Nach Ligation der drei erhaltenen Sequenzen wurde eine PCR mit den Primern:
Felvgag-I (Seq.ID16): CGGATAAGGTACCATGGGCCAAACTATAACTACC
Felvgag-r (Seq.ID17): TTCTCAGAGCTCTTAGAGGAGAGTGGAGTTGGCGGGT
durchgeführt.

Primer für "env" WT:
envl (Seq.ID.18): CGGATAAGGTACCATGGCCAATCCTAGTCCACC
envr (Seq.ID19): AGTTCTCAGAGCTCTTAGGCTGTTTCAAGGAGGGCTT

### Beispiel 2: Kodonoptimierung

Aus der Codon Usage Database (http://www.kazusa.or.ip/codon/) wurde die Kodon Benutzungstabelle für Katzen herausgesucht. Für jede Aminosäure der zwei WT-Sequenzen wurde das Kodon, das am häufigsten für diese Aminosäure kodiert, verwendet. Dabei gab es folgende Einschränkungen:

Wenn eine Aminosäure mehr als 3 mal nacheinander folgte, wurde ab der vierten Aminosäure das zweithäufigste Kodon verwendet. Dadurch sollte die plötzliche extreme Abnahme der tRNA vermieden werden und die Effektivität der Translation gesichert werden.

Um unkontrollierte immunstimulatorische Effekte auszuschließen, wurden Sequenzen mit einem C und einem darauf folgenden G vermieden.

Zur Vermeidung von Schnittstellen von Eco 31I, Kpnl und Sacl wurden alle Sequenzen der Basenfolge GAGCTC, GGTACC, GGTCTC und GAGACC durch Wahl altemativer, ebenfalls häufig benutzter, Kodons entfernt.

### Beispiel 3: Klonierung von FeLVenv

Die Oligonukleotide wurde mit einer Länge zwischen 18 und 28 Basen bestellt (Tip-Molbiol). Insgesamt wurden 51 Oligonukleotide durch Annealing und Ligation miteinander verbunden. Der Überhang betrug 4 Basen. Es wurde darauf geachtet, dass die Überhänge nur einmal vorkamen und nicht palindromisch waren. Jedes einzelne Oligonukleotid wurde mit Strang und Gegenstrang hybridisiert, indem die beiden einzelnen Oligonukleotide (Strang und Gegenstrang) mit Kinasepuffer versetzt auf ca. 80°C erhitzt wurden und dann langsam auf Raumtemperatur abgekühlt wurden. Danach wurde ATP und Polynukleotidkinase (PNK) zugegeben und die Oligonukleotide wurden für eine Stunde phosphoryliert. Danach wurden im ersten Schritt jeweils benachbarte Oligonukleotide zusammengegeben und ligiert (Oligonukleotid 1+2, Oligonukleotid 3+4). Nach 1 h Ligation wurde ein Aliquot von dem Ligationsansatz der Oligonukleotide 1+2 und ein Aliquot vom Ligationsansatz 3+4 genommen und zusammengegeben. Ein Aliquot des letzten Ligationsansatzes wurde genommen und eine PCR mit flankierenden Primem durchgeführt. Entstand ein PCR mit der richtigen erwarteten Länge, wurde es mit TA-Cloning in den TOPO Vektor pCR 2.1 zwischenkloniert und die Sequenz kontrolliert. Dies erfolgte analog für die anderen Fragmente des kompletten Gens. 4 Fragmente wurden erhalten. Die einzelnen Fragmente wurden aus dem zwischenklonierten Plasmid mit Eco RI ausgeschnitten und dann nach Verdau mit Eco 311 mit Ligase ligiert. Das gesamte Ligationsprodukt richtiger Länge wurde mit Bam HI und Sac I verdaut und in den ebenso geschnittenen Vektor nach Gelextraktion in pMCV1.4 kloniert. Anschließend wurde die Sequenz durch Sequenzierung kontrolliert. Das entstandene Plasmid wurde pMCV1.4-FeLVenv genannt.

Primer für die 4 zusammengesetzten Fragmente:
Fragment 1:
   linker Primer (Seq.ID20): ATATTGGATCCCATGGCCAACCCCTCCC
   rechter Primer (Seq.ID21) ATTATGGTCTCCTGCTGCTTCTTCCTGTCTGTGG
Fragment 2:
   linker Primer (Seq.ID22): TAATAGGTCTCCAGCAGCAGACCTACCCCT
   rechter Primer (Seq.ID23): TAATAGGTCTCTGTGAACAGGGCAATGGGGTCA
Fragment 3:
   linker Primer (Seq.ID24): TATTTGGTCTCTTCACAGTGTCCAGGCAGGTGTC
   rechter Primer (Seq.ID25): TATTAGGTCTCAGCTTGTGCTGGGGGGTGG
Fragment 4:
   linker Primer (Seq.ID26): AATAAGGTCTCCAAGCTGACCATCTCTGAGGTGT
   rechter Primer (Seq.ID27): ATTAAGAGCTCTCAGGCTGTTTCCAGC
gesamte Sequenz:
linker Primer (Seq.ID20): ATATTGGATCCCATGGCCAACCCCTCCC
rechter Primer (Seq.ID27): ATTAAGAGCTCTCAGGCTGTTTCCAGC

### Beispiel 4: Klonierungsstrategie für LeadFeLVenv

Zur erfolgreichen Prozessierung des "env" Proteins wurde eine Signalsequenz (Leader Sequenz) vor die kodonoptimierte "env" Sequenz kloniert. Diese Signalsequenz wurde aus insgesamt 8 ODN, mit einer Länge zwischen 22-30 bp, durch Annealing und Ligation hergestellt. Aus dem letzten Ligationsschritt wurde eine PCR zur Amplifizierung der Leadersequenz durchgeführt.

Primer für die gesamte Signalsequenz:
linker Primer (Seq.ID28): ATTGCCGGTACCATGGAGTCCCCCACCCACC
rechter Primer (Seq.ID29): ATCAGAGGTCTCCCATGCCAATGTCAATGGTGAAC

Am 3'-Ende des PCR-Produktes wurde eine Eco31I Erkennungssequenz generiert, die nach Verdau einen Überhang erzeugt, der revers komplementär zu dem am 5'-Ende nach gleichem Verdau erzeugten Überhang des folgenden PCR-Produktes ist.

### PCR für FeLVenv:

Dabei wurde am 5'-Ende der Sequenz eine Eco31I Erkennungssequenz generiert.

Verwendete Primer:
linker Primer (Seq.ID30): GATCTGGGTCTCCATGGCCAACCCCTC
rechter Primer (Seq.ID27): ATTAAGAGCTCTCAGGCTGTTTCCAGC

Nach Verdau der beiden PCR-Produkte mit Eco311 wurden diese gereinigt und miteinander ligiert. Das Ligationsprodukt wurde in eine PCR eingesetzt, bei der eine Erkennungssequenz für Kpnl am 5'-Ende sowie eine für Sacl am 3'-Ende generiert wurden.

Verwendete Primer:
linker Primer (Seq.ID28): ATTGCCGGTACCATGGAGTCCCCCACCCACC
rechter Primer (Seq.ID27): ATTAAGAGCTCTCAGGCTGTTTCCAGC

Das PCR-Produkt wurde mit Kpnl und Sacl verdaut und in den ebenso geschnittenen Vektor pMCV1.4 kloniert. Das entstandene Plasmid wurde pMCV1.4-LeadFeLVenv genannt.

### Beispiel 5: Klonierungsstrategie für LeadFeLVenvgp85

Es wurde das gesamte "env" Polyprotein bestehend aus gp70 und p15 kloniert. Dazu wurde mittels PCR aus dem Plasmid pMCV1.4-FeLVenvp15 die p15 WT Sequenz amplifiziert und hinter die Sequenz des o.a. pMCV1.4-LeadFeLVenv kloniert.

Bei der Amplifizierung des p15 wurde am 5'-Ende eine Eco31I Erkennungssequenz generiert.
1. PCR:
   Verwendete Primer:
      linker Primer (Seq.ID31): AATTATGGTCTCGCAGTTCAGACAACTACAAATGGC
      rechter Primer (Seq.ID32): AATTATGAGCTCTCAGGGCCTGTCAGGGTC
2. PCR:
   Die Sequenz von LeadFeLVenv wurde amplifiziert. Dabei wurde am 3'-Ende eine Eco31 I Erkennungssequenz generiert.
   Verwendete Primer:
   linker Primer (Seq.ID33): AATTATGGTACCATGGAGTCCCCCACCC
   rechter Primer (Seq.ID34): TATAATGGTCTCAACTGGGCTGTTTCCAGCAGGGC

Nach Verdau der beiden PCR-Produkte mit Eco31 I wurden diese miteinander ligiert. Das Ligationsprodukt wurde in eine PCR mit folgenden Primem eingesetzt:
linker Primer (Seq.ID33): AATTATGGTACCATGGAGTCCCCCACCC
rechter Primer (Seq.ID32): AATTATGAGCTCTCAGGGCCTGTCAGGGTC

Dabei wurde am 5'-Ende eine Kpnl Erkennungssequenz, am 3'-Ende eine Sacl Erkennungssequenz generiert. Nach Verdau des PCR-Produktes mit Kpnl und Sacl wurde dieses in den ebenso geschnittenen pMCV1.4 ligiert und kloniert. Das entstandene Plasmid wurde pMCV1.4-LeadFeLVenvgp85 genannt.

### Beispiel 6: Spleiß-Signal Optimierung des LeadFeLVenvgp85 (-spleiß):

Die DNA-Sequenz des LeadFeLVenv wurde unter http://www.fruitfly.org/seq tools/splice.html auf mögliche Spleiß-Signalsequenzen (splice-sites) überprüft. Zwischen den Basen 100 und 140 wurde eine splice-site mit einer 97% Wahrscheinlichkeit erkannt. Nach Austausch der Base 119 (von A nach G = AS-Austausch von Gln nach Arg) wurde keine potentielle splice-site mehr erkannt (untere Grenze = 40% Wahrscheinlichkeit). Die Erzeugung und Klonierung der mutierten Sequenz wurden folgendermaßen durchgeführt:
PCR zur Erzeugung der mutierten Sequenz:
   Zunächst wurde mittels PCR ein Fragment (1), bestehend aus den Basen 1 - 123 des LeadsynFeLVenv amplifiziert. Der verwendete vorwärts-Primer generiert am 5'-Ende des PCR-Produktes die Erkennungssequenz des Restriktionsenzyms Kpnl.
   Die Sequenz des rückwärts-Primer wurde so gewählt, dass das PCR-Produkt die Mutation (Base 119=G) aufweist. Zusätzlich generiert die PCR die Erkennungssequenz des Restriktionsenzyms Eco31I-Site am 3'-Ende des PCR-Produktes. Grundsätzlich erzeugt Eco311 einen 4 Basen 5'-Überhang der Basen 2-5 downstream der Erkennungssequenz.
   Der 4 Basen 5'-Überhang, der nach Verdau des PCR-Produktes mit Eco31I am 3'-Ende des Fragment 1 entsteht, entspricht den Basen 120 - 123 der Sequenz des LeadFeLVenv. Diese Sequenz wiederum entspricht dem Überhang, der auch entsteht, wenn LeadFeLVenv mit dem Restriktionsenzym BgL11 geschnitten wird, da die Basen-1-19-- 1-24-des LeadFeLVenv die Erkennungssequenz von BgLll darstellen.
   Aus dem Konstrukt pMCV1.4-LeadFeLVenv wird somit zunächst mittels Kpnl und BgLII die Basen 1 -123 ausgeschnitten.
   Nach Verdau des PCR-Produktes Fragment 1 (mit Mutation der Base 119 = G) mit Kpnl und Eco31I kann dieses in den mit Kpnl und BgLII geschnittenen und gereinigten pMCV1.4-LeadFeLVenv ligiert und kloniert werden. Das entstandene Plasmid wurde pMCV1.4-FeLVenvgp70 (-spleiß) genannt.
verwendete Primer:
   linker Primer (Seq.ID28): 5'-ATTGCCGGTACCATGGAGTCCCCCACCCACC
   rechter Primer (Seq.ID35): 5'-ATATTAGGTCTCAGATCCGGGGGGGGGAGGG

Analog dazu wurde das PCR-Produkt Fragment 1 in den mit Kpnl und BgLII geschnittenen und gereinigten pMCV1.4-LeadFeLVenvgp85(-spleiß) ligiert und kloniert. Das entstandene Plasmid wurde pMCV1.4-FeLVenvgp85(-spleiß) genannt.

### Beispiel 7: Klonierungsstrategie für FeLVgag:

Die Klonierung von FeLVgag erfolgte entsprechend der unter 3 beschriebenen Vorgehensweise. Die Sequenz wurde über Oligonukleotide, die zunächst zu drei einzelnen Fragmenten annealt und ligiert wurden, hergestellt. Die Sequenz wurde aus insgesamt 2 x 31 Oligonukleotiden (Vorwärts- und Rückwärtsstrang) zusammengesetzt. Die Fragmente wurden als Template in eine PCR eingesetzt und mit folgenden Primem amplifiziert:
Fragment 1:
   linker Primer (Seq.ID36): ATATTGGTCTCAGGAGAGGGACAAGAAGAG
   rechter Primer (Seq.ID37): AATATGGTCTCTCAGCCTGCTGGCGATGGGGC
Fragment 2:
   linker Primer (Seq.ID38): ATTATGGTCTCTGCACCTGAGGCTGTACAGGC
   rechter Primer (Seq.ID39): AATATGGTCTCGGTGCTCCCTGCCGGCGGGGGTGCA
Fragment 3:
   linker Primer (Seq.ID38): ATTATGGTCTCTGCACCTGAGGCTGTACAGGC
   rechter Primer (Seq.ID40): AATATGGTCTCTCTCCTCCTGCCTCTGC
Primer für das gesamte Fragment:
   linker Primer (Seq.ID36): ATATTGGTCTCAGGAGAGGGACAAGAAGAG
   rechter Primer (seq.ID40): AATATGGTCTCTCTCCTCCTGCCTCTGC

Fragment 1, 2 und 3 wurden in den TOPO-Vektor pCR 2.1. zwischenkloniert, anschließend mit Eco 31I verdaut und extrahiert. Das Ligationsprodukt aus 1, 2 und 3 wurde mit Kpnl und Sacl verdaut und in pMCV 1.4 kloniert. Das entstandene Plasmid wurde pCMV1.4-FeLVgag genannt.

### Beispiel 7: Transfektion der Zellen, Expressionsnachweis:

Feline Zellen der f3201 Zellinie wurden mit den Plasmiden, pMCV1.4-FeLVenvgp85(-spleiß), pMCV1.4-FeLVenvgp70(-spleiß), FeLVgag und den WT enthaltenen Plasmiden pMOK für "env" und "gag" mittels Elektroporation bei 250 V und 1050µF transfiziert.

Der Nachweis der exprimierten Proteine wurde mit der Westem Blot Methode geführt. Für den Nachweis dienten monoklonale Mausantikörper. Positiv-Kontrolle: mit FeLV A infizierte Zellen der f3201 Zelllinie.

### Beispiel 8: Herstellung von peptidgekoppelten Midge:

Die Plasmide pMCV1.4-FeLVenvgp85(-spleiß), pMCV1.4-FeLVenvgp70(-spleiß) und pMCV1.4-FeLVgag wurden mit dem Restriktionsenzym Eco311 über Nacht bei 37°C vollständig verdaut. Durch den Restriktionsverdau wurden zwei DNA-Fragmente erzeugt. Das eine bestand aus dem Kanamycin-Resistenzgen, sowie anderen zu Propagierung des Plasmides in Bakterien notwendigen Sequenzen. Das andere Fragment bestand aus den Sequenzen die Bestandteil der Midge-DNA werden sollten: enhanced CMV-Promotor, chimäres Intron, der entsprechenden Gensequenz und der Polyadenylierungssequenz aus SV 40. Mittels des Enzyms T4-DNA-Ligase wurden die 5' phosphorylierten haarnadelförmigen Oligodesoxynukleotide (TIBMolBiol, Berlin) 5' -PH- GGGAGTCCAGT xT TTCTGGAC -3' und 5' PH-AGG GGT CCA GTT TTC TGG AC-3' in Anwesenheit des Restriktionsenzymes Eco31 I über Nacht bei 37°C an das die Midge-DNA bildende DNA Fragment ligiert. Die Reaktion wurde durch Erhitzen auf 70 °C gestoppt. Das resultierende Gemisch an Nukleinsäuren wurde mit dem Enzym T7-DNA-Polymerase behandelt. Die Midge-DNA wurde durch Anionenaustauschchromatographie aufgereinigt und mit Isopropanol gefällt (vgl. EP 0 941 318 B1)

### Herstellung der peptid-gekoppelten ODN:

Das NLS-Peptid PKKKRKV wurde in zwei Schritten an die ODN gekoppelt. Zuerst wurde das modifizierte Oligonukleotid 5'-PH-d(GGGAGTCCAGT xT TTCTGGAC, wobei xT amminomodifizierte Thyminbase mit C₂ - Amminolinker bezeichnet) (0,1mM) mit sulfo-KMUS (5mM) in PBS bei Raumtemperatur (RT) aktiviert. Nach 120 min. wurde die Reaktion mit 50 mM Tris-(Hydroxymethyl)-Aminomethane gestoppt und das aktivierte ODN nach Ethanolpräzipation (300mM NaOAc pH 5.2, 5.5 mM MgCl₂, 70 % Ethanol), Zentrifugation und einmaligem Waschen mit 70% Ethanol erhalten. Das so erhaltene ODN (0,1mM) wurde in PBS gelöst und reagierte mit dem Peptid (0,2mM) für eine Stunde bei Raumtemperatur. Die Reaktion wurde durch Gelektrophorese (3%) und Ethidiumbromidfärbung überprüft. Das entstandene NLS-gekoppelte ODN wurde durch HPLC aufgereinigt und zur Synthese der Midge-NLS Konstrukte wie zuvor beschrieben eingesetzt.

### Beispiel 9: Antikörpertest in Mäusen

Es wurden fünf Impfgruppen zu je sechs BALB/c Mäusen gebildet (siehe Tabelle 1). Grundantigen in allen Gruppen (mit Ausnahme der Kontrollgruppen) waren die optimierten Sequenzen des "env" Proteins mit und ohne immunomodulierendem Protein p15. Die kodierende Sequenz und der Zytomegalo-Virus Promotor (CMV), der der Sequenz vorangestellt ist, werden als lineare Doppelstrangmoleküle entsprechend Bsp.8 verwendet. Als Kontrollen dienten PBS Puffer, herkömmlicher Impfstoff (Leucogen) und der WT des "env" Proteins. Nach der Erstimmunisierung (50 µg DNA, 1mg/ml, i.d.) erfolgte eine Zweitimmunisierung (Boost) am 15. Tag. Blutentnahmen erfolgten am 14., 28. Und 42. Tag. Die Blutproben wurden auf spezifische Antikörper gegen "env" getestet.

**Tabelle 1: Zusammensetzung der Impfgruppen**

| Gr. | Mäuse | Verwendetes Antigen | Fragestellung |
|---|---|---|---|
| 1 | 6 | Leucogen | Positivkontrolle |
| 2 | 6 | PBS-Puffer | Negativkontrolle |
| 3 | 6 | FeLVenvgp85(-spleiß) | Antikörper bestimmen |
| 4 | 6 | FeLVenvgp70(-spleiß) | Antikörper bestimmen |
| 5 | 6 | WT "env" | Positivkontrolle |

Die Ergebnisse sind in Figur 3 gezeigt.

### Beispiel 10a: Immunisierung von Katzen

Zum Testen, ob die synthetischen Sequenzen in der Lage sind, in Katzen eine humorale und zelluläre Immunantwort hervorzurufen, wurde folgendes Impfregime (Tabelle 2) formuliert:

**Tabelle 2: Zusammensetzung der Impfgruppen**

| Gr. | Katzen | Verwendetes Antigen | Fragestellung |
|---|---|---|---|
| 1 | 5 | FeLVenvgp85(-spleiß) FeLVgag | Antikörper-u. Zytokinstatus bestimmen |
| 2 | 5 | FeLVenvgp70(-spleiß) FeLVgag | Antikörper-u. Zytokinstatus bestimmen, Vergleich zu Gruppe 1 |
| 3 | 2 | PBS Puffer | Negativkontrolle |
| 4 | 3 | Leucogen | Positivkontrolle |

Die Katzen der ersten beiden Gruppen werden zweimal mit einer Gesamtmenge von jeweils 600µg DNA, gelöst in PBS Puffer, immunisiert. Die peptidgekoppelten Expressionskonstrukte werden per intradermaler Injektion in den Nacken verabreicht. Die Immunantwort wird über die Dauer von 12 Wochen verfolgt. Die Zweitimmunisierung erfolgt in Woche 4. Durch Bestimmung des Zytokinstatus aus den wöchentlich genommenen Blutproben, sollen Aussagen über die Richtung der Immunantwort (Th1, Th2) getroffen werden. Dabei wurde IL-4 als Indikator einer TH2; IL-2 und Interferon-gamma als Indikator einer vorwiegend TH1 gerichteten Immunantwort bestimmt. Der Impfstoff Leucogen enthält rekombinantes "env" Protein und wird als Positivkontrolle eingesetzt.

Antikörper gegen die eingesetzten Antigene wurden mittels Westernblot und ELISA-Verfahren bestimmt.

Die Menge der mRNA der Zytokine IL-2, IL-4 sowie Interferon-gamma wurde mittels real-time PCR Verfahren bestimmt.

### Beispiel 10b: in vivo Ergebnisse nach Immunisierung von Katzen nach dem in Tabelle 2 beschriebenen Impfregime.

Der semiquantitative Nachweis der Antikörper gegen das "env" Protein erfolgte mittels Westemblot. Getestet wurden Plasmaproben der Katzen aus Versuchswoche 0 und 12. In Woche 0 wurden erwartungsgemäß keine Antikörper gegen das "env" nachgewiesen. Bei den in der Tabelle 3 aufgeführten schwachen Banden (+) handelt es sich um unspezifische Banden. In Woche 12 zeigten alle Tiere der Gruppen 1, 2 und 4 mit Ausnahme einer Katze eine deutliche Antikörperantwort. Diese Ergebnisse in der Zieltierart bestätigen jene aus dem Vorversuch in Mäusen (vgl. Beispiel 9).

Dabei bedeuten:
- +++: sehr starke Bande,
- ++: starke Bande,
- +: sichtbare Bande,
- (+): schwache Bande.

Die Stärke der Banden repräsentieren die Konzentration der Antikörper im Plasma der immunisierten Katzen.

**Tabelle 3: Bestimmung der humoralen Immunantwort gegen FeLVenv-Protein**

| Gr. | Verwendetes Antigen | Katzen | Woche 0 | Woche 12 |
|---|---|---|---|---|
| 1 | FeLVenvgp85(-spleiß) | 1 | - | +++ |
| | FeLVgag | 2 | - | ++ |
| | | 3 | - | ++ |
| | | 4 | - | ++ |
| | | 5 | - | ++ |
| 2 | FeLVenvgp70(-spleiß) | 1 | - | +++ |
| | FeLVgag | 2 | (+) | +++ |
| | | 3 | - | ++ |
| | | 4 | - | ++ |
| | | 5 | - | + |
| 3 | PBS Puffer | 1 | (+) | - |
| | | 2 | (+) | (+) |
| 4 | Leucogen | 1 | - | +++ |
| | | 2 | - | +++ |
| | | 3 | - | (+) |

### Beispiel 11: Überprüfung des Impfschutzes durch Belastungsinfektion

Um zu überprüfen, ob die erreichte hohe Antikörperproduktion auch tatsächlich zu einem Schutz gegen die Infektion mit dem FeL-Virus in der Lage ist und somit die Wirksamkeit des erfindungsgemäßen Impfstoffes überprüft werden konnte, schloss sich ein Experiment mit Belastungsinfektion an. Dafür wurden vier Gruppen mit jeweils 10 Katzen zweimal (Tag 0 und Tag 21) mit den entsprechenden Konstrukten intradermal mit einem nadelfreien Injektionsgerät geimpft (Tabelle 4). Als Expressionskonstrukte wurden beschriebene mit dem NLS-Peptid gekoppelte Midge Vektoren eingesetzt. Am Tag 21, 22 und 23 nach der letzten Impfung wurden die Katzen mit lebendem FeL-Virus (Rickard Stamm, > 10e6 focus-forming units/ml) durch eine Belastungsinfektion infiziert. Die Wirksamkeit des Impfstoffes wurde danach beurteilt, ob die Katzen nach der Belastungsinfektion geschützt waren. Als geschützt gelten Katzen, die keine Viruspartikel im Serum (seronegative Katzen) und keine Virus-DNA in ihren Blutzellen aufweisen. Zum Nachweis von Viruspartikeln wurde das Katzenserum auf Vorhandensein des Antigens p27 mittels ELISA getestet. Die Menge an integrierter Virus-DNA, die sogenannte Provirus-DNA, wurde mittels eines Taqman PCR Verfahrens überprüft. Folgendes Impfregime wurde formuliert:

**Tabelle 4:**

| Gr. | Verwendetes Antigen | DNA-Dosis [µg] | Anzahl seronegativer Katzen 105 Tage nach der Belastungsinfektion |
|---|---|---|---|
| 1 | PBS Puffer | - | 0 |
| 2 | FeLVgag | 2 x 100 | 2 |
| 3 | FeLVenvgp70(-spleiß) | 2 x 50 | 4 |

Als Negativkontrolle wurde PBS Puffer eingesetzt.

Die Ergebnisse können wie folgt zusammengefasst werden:

Tabelle 4 zeigt die Ergebnisse der Serumuntersuchung der Katzen hinsichtlich des Vorhandenseins des Virusproteins p27. Dieser Test ist ein allgemein anerkannter Test, um die FeLV Virämie zu diagnostizieren. Parallel dazu wurden weiße Blutzellen derselben Katzen auf das Vorhandensein Proviraler-DNA getestet (Daten nicht gezeigt). Alle Virusprotein-freien Katzen waren auch frei von Proviraler-DNA.

Da beide Testsysteme unterschiedliche Stadien der Virusentwicklung im Körper nachweisen, kann bei zweifachem negativen Ergebnis davon ausgegangen werden, das sich das Virus nicht im Körper der Tiere vermehren konnte, was mit einem erreichten Schutz gleichzusetzen ist.

In den Gruppen 2 und 3 konnte ein Teil der Katzen gegen die Infektion mit FeLV geschützt werden.

In Gruppe 2 waren 2 von 10 Katzen sowohl frei von Virusprotein als auch frei von proviraler DNA. Dieser erreichte Impfschutz beruhte auf die Verabreichung des erfindungsgemäßen Impfstoffs (Seq.ID 5).

In der Gruppe 3 konnten 40% der Tiere mit dem erfindungsgemäßen Impfstoff (Seq. ID 8) vor der Infektion mit dem FeL-Virus geschützt werden. Das ist eine signifikante Reduktion infizierter Katzen im Vergleich zu Gruppe 1.

Bei den Tieren konnte ebenfalls weder Virusprotein noch Provirus-DNA in den Serum-und Blutproben nachgewiesen werden. Bemerkenswert ist, das zum Schutz der 4 Katzen in Gruppe 3 die sehr geringe Dosis von 50µg je Injektion ausreichend war, um die Katzen zu schützen. Das ist von Vorteil, da die zu verabreichende DNA-Konzentration gering ist und somit die Herstellungskosten des Impfstoffes rapide gesenkt werden.

Alle Tiere der Gruppe 1 (Kontrollgruppe) zeigten Viruspartikel im Blut, d.h., sie waren nicht geschützt und voll empfänglich für die Belastungsinfektion.

Während des gesamten Immunisierungsversuchs wurden weder Nebenwirkungen inform von lokalen Reizungen an den Injektionsstellen, noch Störungen des Allgemeinbefindens der Versuchtiere beobachtet.

## Patentansprüche

1. DNA-Expressionskonstrukt zur Expression von Genprodukten des Felinen Leukosevirus (FeLV) in Katzenzellen, bestehend aus einer in Feliden operablen Promotorsequenz und mindestens einer Nukleotidsequenz, die für das Strukturprotein "gag" und das Membranprotein "env" des FeLV gemäß Seq.ID6 und Seq.ID9 oder gemäß Seq.ID6 und Seq.ID10 kodiert.

2. DNA-Expressionskonstrukt nach Anspruch 1, enthaltend die für das mutagenisierte "gag" Strukturprotein kodierende Nukleotidsequenz Seq.ID5.

3. DNA-Expressionskonstrukt nach Anspruch 1, enthaltend die für das mutagenisierte env-gp85 Membranprotein kodierende Nukleotidsequenz Seq.ID7.

4. DNA-Expressionskonstrukt nach Anspruch 1, enthaltend die für das mutagenisierte env-gp70 Membranprotein kodierende Nukleotidsequenz Seq.ID8.

5. DNA-Expressionskonstrukt nach wenigstens einem der Ansprüche 2 - 4, wobei die angegebenen Nukleotidsequenzen vollständig enthalten sind und jeweils für ein gesamtes Struktur- und/oder Membranprotein kodieren.

6. DNA-Expressionskonstrukt nach wenigstens einem der Ansprüche 1 bis 5, wobei das Expressionskonstrukt ein Plasmid ist.

7. DNA-Expressionskonstrukt nach wenigstens einem der Ansprüche 1 bis 5, wobei die immunisierenden Polynukleotidsequenzen als Expressionskonstrukte vorliegen, die aus kovalent geschlossenen linearen Desoxyribonukleinsäuremolekülen bestehen, welche einen linearen Doppelstrangbereich aufweisen und wobei die doppelstrangbildenden Einzelstränge durch kurze einzelsträngige Schlaufen aus Desoxyribonukleinsäurenukleotiden verknüpft sind, und wobei die doppelstrangbildenden Einzelstränge nur aus der kodierenden Sequenz unter Kontrolle eines im zu impfenden Tier operablen Promotors und einer Terminatorsequenz besteht.

8. DNA-Expressionskonstrukt nach wenigstens einem der vorhergehenden Ansprüche, wobei das Expressionskonstrukt mit einem oder mehreren Peptiden kovalent verknüpft ist.

9. DNA-Expressionskonstrukt nach Anspruch 8, wobei das Peptid aus 3 bis 30 Aminosäuren besteht, von denen mindestens die Hälfte basische Aminosäuren aus der Gruppe Arginin und Lysin kommen.

10. DNA-Expressionskonstrukt nach Anspruch 9, wobei das Peptid die Aminosäuresequenz PKKKRKV (Potin-Lysin-Lysin-Lysin-Arginin-Lysin-Valin) aufweist.

11. Arzneimittel, insbesondere eine Vakzine, zur Erzeugung einer präventiven und/oder therapeutischen Immunität bei Feliden, insbesondere der Katze, enthaltend ein DNA-Expressionskonstrukt nach einem oder mehreren der Ansprüche 1 bis 10.

12. Protein mit der Aminosäuresequenz gemäß Seq.ID6.

13. Protein mit der Aminosäuresequenz gemäß Seq.ID9.

14. Protein mit der Aminosäuresequenz gemäß Seq.ID10.

## Claims

1. DNA expression construct for the expression of gene products of the Feline Leucosis Virus (FeLV) in cat cells, comprising a promoter sequence that is operable in Felidae, and at least one nucleotide sequence encoding for the structural protein "gag" and the membrane protein "env" of FeLV according to Seq.ID6 and Seq.ID9 or according to Seq.ID6 and Seq.ID10.

2. DNA expression construct according to claim 1, comprising mutagenized nucleotide sequence Seq.ID5 encoding "gag" structural protein.

3. DNA expression construct according to claim 1, comprising mutagenized nucleotide sequence Seq.ID7 encoding env-gp85 membrane protein.

4. DNA expression construct according to claim 1, comprising mutagenized nucleotide sequence Seq.ID8 encoding env-gp70 membrane protein.

5. DNA expression construct according to at least one of the preceding claims, where the indicated nucleotide sequences are comprised entirely encoding for a whole structural and /or membrane protein.

6. DNA expression construct according to at least one of the claims 1 to 5, where the expression construct is a plasmid.

7. DNA expression construct according to at least one of the claims 1 to 5, where the immunizing polynucleotide sequences are in the form of expression constructs consisting of covalently closed linear deoxyribonucleotide molecules comprising a linear double stranded region and where the single strands forming the double strand are linked by short single stranded loops consisting of deoxyribonucleotides, and where said double strand forming single strands only consist of encoding sequence under control of a promoter that is operable in the animal that is to be vaccinated, and a terminator sequence.

8. DNA expression construct according to at least one of the preceding claims, where the expression construct is covalently linked to one or more peptides.

9. DNA expression construct according to claim 8, where the peptide is composed of three to 30 amino acids, at least half of which are a member of the group consisting of arginine and lysine.

10. DNA expression construct according to claim 9, where the peptide comprises the sequence PKKKRKV (proline - lysine - lysine - lysine - arginine - lysine - valine).

11. Pharmaceutical composition, especially a vaccine, for the production of a preventive and /or therapeutic immunity in Felidae, especially the cat, comprising a DNA expression construct according to one or several of the claims 1 to 10.

12. Protein with the amino acid sequence according to Seq.ID6.

13. Protein with the amino acid sequence according to Seq.ID9.

14. Protein with the amino acid sequence according to Seq.ID10.

## Revendications

1. Construct d'expression d'ADN pour l'expression de produits génétiques du virus de la leucose féline (FeLV) dans des cellules de chat, composé d'une séquence promoteur exploitable chez les félidés, et d'au moins une séquence nucléotidique qui code pour la protéine de structure « gag » et la protéine membranaire « env » du FeLV selon l'ID seq. 6 et l'ID seq. 9 ou selon l'ID seq. 6 et l'ID seq. 10.

2. Construct d'expression d'ADN selon la revendication 1, contenant la séquence nucléotidique ID seq. 5 codant pour la protéine de structure « gag » mutagénisée.

3. Construct d'expression d'ADN selon la revendication 1, contenant la séquence nucléotidique ID seq. 7 codant pour la protéine membranaire env-gp85 mutagénisée.

4. Construct d'expression d'ADN selon la revendication 1, contenant la séquence nucléotidique ID seq. 8 codant pour la protéine membranaire env-gp70 mutagénisée.

5. Construct d'expression d'ADN selon au moins l'une des revendications 2 à 4, dans lequel les séquences nucléotidiques indiquées sont contenues entièrement et codent respectivement pour une protéine de structure et/ou membranaire complète.

6. Construct d'expression d'ADN selon au moins l'une des revendications 1 à 5, dans lequel le construct d'expression est un plasmide.

7. Construct d'expression d'ADN selon au moins l'une des revendications 1 à 5, dans lequel les séquences polynucléotidiques immunisantes se présentent en tant que constructs d'expression qui se composent de molécules d'acide désoxyribonucléique linéaires fermées de façon covalente qui présentent une zone bicaténaire linéaire, et dans lequel les simples brins formant le double brin sont reliés par de courtes boucles monocaténaires de nucléotides d'acide désoxyribonucléique, et dans lequel les simples brins formant le double brin se composent uniquement de la séquence codante sous le contrôle d'un promoteur exploitable dans l'animal à inoculer et d'une séquence terminateur.

8. Construct d'expression d'ADN selon au moins l'une des revendications précédentes, dans lequel le construct d'expression est relié de façon covalente à un ou plusieurs peptides.

9. Construct d'expression d'ADN selon la revendication 8, dans lequel le peptide se compose de 3 à 30 acides aminés, parmi lesquels au moins la moitié proviennent d'acides aminés basiques issus du groupe de l'arginine et de la lysine.

10. Construct d'expression d'ADN selon la revendication 9, dans lequel le peptide présente la séquence d'acides aminés PKKKRKV (proline-lysine-lysine-lysine-arginine-lysine-valine).

11. Médicament, en particulier vaccin, permettant de générer une immunité préventive et/ou thérapeutique chez les félidés, en particulier le chat, contenant un construct d'expression d'ADN selon une ou plusieurs des revendications 1 à 10.

12. Protéine ayant la séquence d'acides aminés selon l'ID seq. 6.

13. Protéine ayant la séquence d'acides aminés selon l'ID seq. 9.

14. Protéine ayant la séquence d'acides aminés selon l'ID seq. 10.


### SEQUENCE LISTING

<110> Mologen Forschungs-, Entwicklungs- und vertriebs GmbH
<120> Impfstoff gegen Infektionen mit onkoviren, wie dem felinen Leukosevirus der Katze
<130> XI 1292-03
<150> DE 102 44 863.9
   <151> 2002-09-23
<160> 40
<170> PatentIn version 3.1
<210> 1
   <211> 1929
   <212> DNA
   <213> Feline leukemia virus
<220>
   <221> gene
   <222> (1)..(1929)
   <223> DNA sequence wild type "env" gene without signal peptide coding r egion
<300>
   <308> NCBI M12500
   <309> 2001-02-21
   <313> (162)..(1990)
<400> 1
   atggaaagtc caacgcaccc aaaaccctct aaagataaga ctctctcgtg gaacttagcg      60
   tttctggtgg ggatcttatt tacaatagac ataggaatgg ccaatcctag tccacaccaa     120
   atatataatg taacttgggt aataaccaat gtacaaacta acacccaagc taacgccacc     180
   tctatgttag gaaccttaac cgatgcctac cctaccctac atgttgactt atgtgaccta    240
   gtgggagaca cctgggaacc tatagtccta aacccaacca atgtaaaaca cggggcacgt    300
   tactcctcct caaaatatgg atgtaaaact acagatagaa aaaaacagca acagacatac    360
   cccttttacg tctgccccgg acatgccccc tcgttggggc caaagggaac acattgtgga     420
   ggggcacaag atgggttttg tgccgcatgg ggatgtgaga ccaccggaga agcttggtgg    480
   aagcccacct cctcatggga ctatatcaca gtaaaaagag ggagtagtca ggacaatagc    540
   tgtgagggaa aatgcaaccc cctggttttg cagttcaccc agaagggaag acaagcctct    600
   tgggacggac ctaagatgtg gggattgcga ctataccgta caggatatga ccctatcgct    660
   ttattcacgg tgtcccggca ggtatcaacc attacgccgc ctcaggcaat gggaccaaac    720
   ctagtcttac ctgatcaaaa acccccatcc cgacaatctc aaacagggtc caaagtggcg    780
   acccagaggc cccaaacgaa tgaaagcgcc ccaaggtctg ttgcccccac caccatgggt    840
   cccaaacgga ttgggaccgg agataggtta ataaatttag tacaagggac atacctagcc    900
   ttaaatgcca ccgaccccaa caaaactaaa gactgttggc tctgcctggt ttctcgacca    960
   ccctattacg aagggattgc aatcttaggt aactacagca accaaacaaa ccccccccca    1020
   tcctgcctat ctactccgca acacaaacta actatatctg aagtatcagg gcaaggaatg   1080
   tgcataggga ctgttcctaa aacccaccag gctttgtgca ataagacaca acagggacat   1140
   acaggggcgc actatctagc cgcccccaac ggcacctatt gggcctgtaa cactggactc   1200
   accccatgca tttccatggc ggtgctcaat tggacctctg atttttgtgt cttaatcgaa   1260
   ttatggccca gagtgactta ccatcaaccc gaatatgtgt acacacattt tgccaaagct   1320
   gtcaggttcc gaagagaacc aatatcacta acggttgccc ttatgttggg aggacttact   1380
   gtagggggca tagccgcggg ggtcggaaca gggactaaag ccctccttga aacagcccag   1440
   ttcagacaac tacaaatggc catgcacaca gacatccagg ccctagaaga atcaattagt   1500
   gccttagaaa agtccctgac ctccctttct gaagtagtct tacaaaacag acggggccta   1560
   gatattctat tcttacaaga gggagggctc tgtgccgcat tgaaagaaga atgttgcttc   1620
   tatgcggatc acaccggact cgtccgagac aatatggcca aattaagaga aagactaaaa   1680
   cagcggcaac aactgtttga ctcccaacag ggatggtttg aaggatggtt caacaagtcc   1740
   ccctggttta caaccctaat ttcctccatt atgggcccct tactaatcct actcctaatt   1800
   ctcctcttcg gcccatgcat ccttaaccga ttagtacaat tcgtaaaaga cagaatatct   1860
   gtggtacagg ctttaatttt aacccaacag taccaacaga taaagcaata cgatccggac   1920
   cgaccatga                                                            1929
<210> 2
   <211> 1527
   <212> DNA
   <213> Feline leukemia virus
<220>
   <221> gene
   <222> (1)..(1527)
   <223> DNA sequence wild type "gag" gene
<400> 2
   atgggccaaa ctataactac ccccttgagc ctcaccctca accactggtc tgaggttcag      60
   gcacgggccc gtaatcaggg tgtcgaagtc cggaaaaaga aatggattac actgtgtgaa     120
   gccgaatggg taatgatgaa tgtaggttgg ccccgagaag gaactttcac cattgacaat     180
   atttcacagg tcgaggagag aatcttcgcc ccggggccat atggacaccc agatcaaatc     240
   ccttatatta ccacgtggag atccctagcc acagaccccc ctccatgggt tcgcccattc     300
   ctaccccctc ctaagcatcc caggacagat cctcccgagc ctctttcgcc gcaacctctt     360
   gcgccgcaac cctcttcccc ccaccccgtc ctctaccccg ttctccccaa accagacccc     420
   cccaaggcgc ctgtattacc acccaatcct tcttcccctt taattgatct cttaacagaa     480
   gagccacctc cctatcctgg gggtcacggg ccaacaccgc cgtcaggccc tagaacccca     540
   actgcctccc cgattgccat ccggctgcga gaacgacgag aaaatccagc tgagaaatct     600
   caagccctcc ccttaaggga agacccaaac aacagacccc agtactggcc attctcggcc     660
   tctgacctgt acaattggaa attgcataac ccccctttct cccaggaccc agtggcccta     720
   actaacctaa ttgagtccat tttagtgaca catcagccaa cctgggacga ctgccaacag     780
   ctcttacagg ctctcctgac ggcagaggag agacaaaggg tcctccttga agcccgaaag     840
   caagttccag gcgaggacgg acggccaacc cagctgccca atgtcgttga cgaggctttc     900
   cccttgaccc gtcccaactg ggatttttgt acgccggcag gtagggagca cctacgcctt     960
   tatcgccagt tgctgttagc ggggctccgc ggggctgcaa gacgccccac taatttggca    1020
   caggtaaagc aagttgtaca agggaaagag gaaacgccag cctcattctt agaaagatta    1080
   aaagaggctt acagaatgta tactccctat gaccctgagg acccagggca ggctgctagt    1140
   gttatcctgt cctttatcta ccagtctagc ccggacataa gaaataagtt acaaaggcta    1200
   gaaggcctac aggggttcac actgtctgat ttgctaaaag aggcagaaaa gatatacaac    1260
   aaaagggaaa ccccagagga aagggaagaa agattatggc agcggcagga agaaagagat    1320
   aaaaagcgcc ataaggagat gactaaagtt ctggccacag tagttgctca gaatagagat    1380
   aaggatagag gggaaagtaa actgggagat caaaggaaaa tacctctggg gaaagaccag    1440
   tgtgcctatt gcaaggaaaa gggacattgg gttcgcgatt gcccgaaacg accccggaag    1500
   aaacccgcca actccactct cctctaa                                        1527
<210> 3
   <211> 642
   <212> PRT
   <213> Feline leukemia virus
<220>
   <221> PEPTIDE
   <222> (1)..(447)
   <223> Amino acid sequence of the protein corresponding to Seq.ID1
<400> 3
   Met Glu Ser Pro Thr His Pro Lys Pro Ser Lys Asp Lys Thr Leu Ser
   1               5                   10                  15
   Trp Asn Leu Ala Phe Leu Val Gly Ile Leu Phe Thr Ile Asp Ile Gly
               20                  25                  30
   Met Ala Asn Pro Ser Pro His Gln Ile Tyr Asn Val Thr Trp Val Ile
           35                  40                  45
   Thr Asn Val Gln Thr Asn Thr Gln Ala Asn Ala Thr Ser Met Leu Gly
       50                  55                  60
   Thr Leu Thr Asp Ala Tyr Pro Thr Leu His Val Asp Leu Cys Asp Leu
   65                  70                  75                  80
   Val Gly Asp Thr Trp Glu Pro Ile Val Leu Asn Pro Thr Asn Val Lys
                   85                  90                  95
   His Gly Ala Arg Tyr Ser Ser Ser Lys Tyr Gly Cys Lys Thr Thr Asp
               100                 105                 110
   Arg Lys Lys Gln Gln Gln Thr Tyr Pro Phe Tyr Val Cys Pro Gly His
           115                 120                 125
   Ala Pro Ser Leu Gly Pro Lys Gly Thr His Cys Gly Gly Ala Gln Asp
       130                 135                 140
   Gly Phe Cys Ala Ala Trp Gly Cys Glu Thr Thr Gly Glu Ala Trp Trp
   145                 150                 155                 160
   Lys Pro Thr Ser Ser Trp Asp Tyr Ile Thr Val Lys Arg Gly Ser Ser
                   165                 170                 175
   Gln Asp Asn Ser Cys Glu Gly Lys Cys Asn Pro Leu Val Leu Gln Phe
               180                 185                 190
   Thr Gln Lys Gly Arg Gln Ala Ser Trp Asp Gly Pro Lys Met Trp Gly
   195 200 205
   Leu Arg Leu Tyr Arg Thr Gly Tyr Asp Pro Ile Ala Leu Phe Thr Val
   210 215 220
   Ser Arg Gln Val Ser Thr Ile Thr Pro Pro Gln Ala Met Gly Pro Asn
   225 230 235 240
   Leu Val Leu Pro Asp Gln Lys Pro Pro Ser Arg Gln Ser Gln Thr Gly
   245 250 255
   Ser Lys Val Ala Thr Gln Arg Pro Gln Thr Asn Glu Ser Ala Pro Arg
   260 265 270
   Ser Val Ala Pro Thr Thr Met Gly Pro Lys Arg Ile Gly Thr Gly Asp
   275 280 285
   Arg Leu Ile Asn Leu Val Gln Gly Thr Tyr Leu Ala Leu Asn Ala Thr
   290 295 300
   Asp Pro Asn Lys Thr Lys Asp Cys Trp Leu Cys Leu Val Ser Arg Pro
   305 310 315 320
   Pro Tyr Tyr Glu Gly Ile Ala Ile Leu Gly Asn Tyr Ser Asn Gln Thr
   325 330 335
   Asn Pro Pro Pro Ser Cys Leu Ser Thr Pro Gln His Lys Leu Thr Ile
   340 345 350
   Ser Glu Val Ser Gly Gln Gly Met Cys Ile Gly Thr Val Pro Lys Thr
   355 360 365
   His Gln Ala Leu Cys Asn Lys Thr Gln Gln Gly His Thr Gly Ala His
   370 375 380
   Tyr Leu Ala Ala Pro Asn Gly Thr Tyr Trp Ala Cys Asn Thr Gly Leu
   385 390 395 400
   Thr Pro Cys Ile Ser Met Ala Val Leu Asn Trp Thr Ser Asp Phe Cys
   405 410 415
   Val Leu Ile Glu Leu Trp Pro Arg Val Thr Tyr His Gln Pro Glu Tyr
   420 425 430
   Val Tyr Thr His Phe Ala Lys Ala Val Arg Phe Arg Arg Glu Pro Ile
   435 440 445
   Ser Leu Thr Val Ala Leu Met Leu Gly Gly Leu Thr Val Gly Gly Ile
   450 455 460
   Ala Ala Gly Val Gly Thr Gly Thr Lys Ala Leu Leu Glu Thr Ala Gln
   465 470 475 480
   Phe Arg Gln Leu Gln Met Ala Met His Thr Asp Ile Gln Ala Leu Glu
   485 490 495
   Glu Ser Ile Ser Ala Leu Glu Lys Ser Leu Thr Ser Leu Ser Glu Val
   500 505 510
   Val Leu Gln Asn Arg Arg Gly Leu Asp Ile Leu Phe Leu Gln Glu Gly
   515 520 525
   Gly Leu Cys Ala Ala Leu Lys Glu Glu Cys Cys Phe Tyr Ala Asp His
   530 535 540
   Thr Gly Leu Val Arg Asp Asn Met Ala Lys Leu Arg Glu Arg Leu Lys
   545 550 555 560
   Gln Arg Gln Gln Leu Phe Asp Ser Gln Gln Gly Trp Phe Glu Gly Trp
   565 570 575
   Phe Asn Lys Ser Pro Trp Phe Thr Thr Leu Ile Ser Ser Ile Met Gly
   580 585 590
   Pro Leu Leu Ile Leu Leu Leu Ile Leu Leu Phe Gly Pro Cys Ile Leu
   595 600 605
   Asn Arg Leu Val Gln Phe Val Lys Asp Arg Ile Ser Val Val Gln Ala
   610 615 620
   Leu Ile Leu Thr Gln Gln Tyr Gln Gln Ile Lys Gln Tyr Asp Pro Asp
   625 630 635 640
   Arg Pro
<210> 4
   <211> 508
   <212> PRT
   <213> Feline leukemia virus
<220>
   <221> PEPTIDE
   <222> (1)..(508)
   <223> Amino acid sequence of the protein corresponding to seq.ID2
<400> 4
   Met Gly Gln Thr Ile Thr Thr Pro Leu Ser Leu Thr Leu Asn His Trp
   1               5                   10                  15
   Ser Glu Val Gln Ala Arg Ala Arg Asn Gln Gly Val Glu Val Arg Lys
               20                  25                  30
   Lys Lys Trp Ile Thr Leu Cys Glu Ala Glu Trp Val Met Met Asn Val
           35                  40                  45
   Gly Trp Pro Arg Glu Gly Thr Phe Thr Ile Asp Asn Ile Ser Gln Val
       50                  55                  60
   Glu Glu Arg Ile Phe Ala Pro Gly Pro Tyr Gly His Pro Asp Gln Ile
   65                  70                  75                  80
   Pro Tyr Ile Thr Thr Trp Arg Ser Leu Ala Thr Asp Pro Pro Pro Trp
                   85                  90                  95
   Val Arg Pro Phe Leu Pro Pro Pro Lys His Pro Arg Thr Asp Pro Pro
               100                 105                 110
   Glu Pro Leu Ser Pro Gln Pro Leu Ala Pro Gln Pro Ser Ser Pro His
           115                 120                 125
   Pro Val Leu Tyr Pro Val Leu Pro Lys Pro Asp Pro Pro Lys Ala Pro
       130                 135                 140
   Val Leu Pro Pro Asn Pro Ser Ser Pro Leu Ile Asp Leu Leu Thr Glu
   145                 150                 155                 160
   Glu Pro Pro Pro Tyr Pro Gly Gly His Gly Pro Thr Pro Pro Ser Gly
                   165                 170                 175
   Pro Arg Thr Pro Thr Ala Ser Pro Ile Ala Ile Arg Leu Arg Glu Arg
               180                 185                 190
   Arg Glu Asn Pro Ala Glu Lys Ser Gln Ala Leu Pro Leu Arg Glu Asp
           195                 200                 205
   Pro Asn Asn Arg Pro Gln Tyr Trp Pro Phe Ser Ala Ser Asp Leu Tyr
       210                 215                 220
   Asn Trp Lys Leu His Asn Pro Pro Phe Ser Gln Asp Pro Val Ala Leu
   225                 230                 235                 240
   Thr Asn Leu Ile Glu Ser Ile Leu Val Thr His Gln Pro Thr Trp Asp
                   245                 250                 255
   Asp Cys Gln Gln Leu Leu Gln Ala Leu Leu Thr Ala Glu Glu Arg Gln
               260                 265                 270
   Arg Val Leu Leu Glu Ala Arg Lys Gln Val Pro Gly Glu Asp Gly Arg
           275                 280                 285
   Pro Thr Gln Leu Pro Asn Val Val Asp Glu Ala Phe Pro Leu Thr Arg
       290                 295                 300
   Pro Asn Trp Asp Phe Cys Thr Pro Ala Gly Arg Glu His Leu Arg Leu
   305                 310                 315                 320
   Tyr Arg Gln Leu Leu Leu Ala Gly Leu Arg Gly Ala Ala Arg Arg Pro
                   325                 330                 335
   Thr Asn Leu Ala Gln Val Lys Gln Val Val Gln Gly Lys Glu Glu Thr
               340                 345                 350
   Pro Ala Ser Phe Leu Glu Arg Leu Lys Glu Ala Tyr Arg Met Tyr Thr
           355                 360                 365
   Pro Tyr Asp Pro Glu Asp Pro Gly Gln Ala Ala Ser Val Ile Leu Ser
       370                 375                 380
   Phe Ile Tyr Gln Ser Ser Pro Asp Ile Arg Asn Lys Leu Gln Arg Leu
   385                 390                 395                 400
   Glu Gly Leu Gln Gly Phe Thr Leu Ser Asp Leu Leu Lys Glu Ala Glu
                   405                 410                 415
   Lys Ile Tyr Asn Lys Arg Glu Thr Pro Glu Glu Arg Glu Glu Arg Leu
               420                 425                 430
   Trp Gln Arg Gln Glu Glu Arg Asp Lys Lys Arg His Lys Glu Met Thr
           435                 440                 445
   Lys Val Leu Ala Thr Val Val Ala Gln Asn Arg Asp Lys Asp Arg Gly
       450                 455                 460
   Glu Ser Lys Leu Gly Asp Gln Arg Lys Ile Pro Leu Gly Lys Asp Gln
   465                 470                 475                 480
   Cys Ala Tyr Cys Lys Glu Lys Gly His Trp Val Arg Asp Cys Pro Lys
                   485                 490                 495
   Arg Pro Arg Lys Lys Pro Ala Asn Ser Thr Leu Leu
               500                 505
<210> 5
   <211> 1530
   <212> DNA
   <213> Feline leukemia virus
<220>
   <221> misc_feature
   <222> (1)..(1530)
   <223> DNA sequence of the mutagenized "gag" gene
<400> 5
   atgggccaga ccatcaccac ccccctgagc ctgaccctga accactggag cgaggtgcag      60
   gccagggcca ggaaccaggg cgtggaggtg aggaagaaga agtggatcac cctgtgcgag     120
   gccgagtggg tgatgatgaa cgtgggctgg cccagggagg gcaccttcac catcgacaac     180
   atcagccagg tggaggagag gatcttcgcc cccggcccct acggccaccc cgaccagatc     240
   ccctacatca ccacctggag gagcctggcc accgaccccc ccccctgggt gaggcccttc    300
   ctgccccccc ccaagcaccc caggaccgac ccccccgagc ccctgagccc ccagcccctg   360
   gccccccagc ccagcgcccc ccccatcagc agcctgtacc ccgtgctgcc caagcccgac     420
   ccccccaagg cccccgtgct gccccccaac cccagcagcc ccctgatcga cctgctgacc   480
   gaggagcccc ccccctaccc cggcggccac ggccccaccc cccccagcgg ccccaggacc   540
   cccaccgcca gccccatcgc cagcaggctg agggagagga gggagaaccc cgccgagaag     600
   agccaggccc tgcccctgag ggaggacccc aacaacaggc cccagtactg gcccttcagc     660
   gccagcgacc tgtacaactg gaagctgcac aaccccccct tcagccagga ccccgtggcc     720
   ctgaccaacc tgatcgagag catcctggtg acccaccagc ccacctggga cgactgccag     780
   cagctgctgc aggccctgct gaccgccgag gagaggcaga gggtgctgct ggaggccagg     840
   aagcaggtgc ccggcgagga cggcaggccc acccagctgc ccaacgtggt ggacgaggcc     900
   ttccccctga ccaggcccaa ctgggacttc tgcacccccg ccggcaggga gcacctgagg   960
   ctgtacaggc agctgctgct ggccggcctg aggggcgccg ccaggaggcc caccaacctg    1020
   gcccaggtga agcaggtggt gcagggcaag gaggagacac ccgccagctt cctggagagg    1080
   ctgaaggagg cctacaggat gtacaccccc tacgaccccg aggaccccgg ccaggccacc    1140
   agcgtgatcc tgagcttcat ctaccagagc agccccgaca tcaggaacaa gctgcagagg    1200
   ctggagggcc tgcagggctt caccctgagc gacctgctga aggaggccga gaagatctac    1260
   aacaagaggg agacacccga ggagagggag gagaggctgt ggcagaggca ggaggagagg    1320
   gacaagaaga ggcacaagga gatgaccaag gtgctggcca ccgtggtggc ccagaacagg    1380
   gacaaggaca ggggcgagag caagctgggc gaccagagga agatccccct gggcaaggac  1440
   cagtgcgcct actgcaagga gaagggccac tgggtgaggg actgccccaa gaggcccagg    1500
   aagaagcccg ccaacagcac cctgctgtag                                     1530
<210> 6
   <211> 509
   <212> PRT
   <213> Feline leukemia virus
<220>
   <221> PEPTIDE
   <222> (1)..(509)
   <223> Amino acid sequence of the protein corresponding to seq.ID5
<400> 6
   Met Gly Gln Thr Ile Thr Thr Pro Leu Ser Leu Thr Leu Asn His Trp
   1               5                   10                  15
   Ser Glu Val Gln Ala Arg Ala Arg Asn Gln Gly Val Glu Val Arg Lys
               20                  25                  30
   Lys Lys Trp Ile Thr Leu Cys Glu Ala Glu Trp Val Met Met Asn Val
           35                  40                  45
   Gly Trp Pro Arg Glu Gly Thr Phe Thr Ile Asp Asn Ile Ser Gln Val
       50                  55                  60
   Glu Glu Arg Ile Phe Ala Pro Gly Pro Tyr Gly His Pro Asp Gln Ile
   65                  70                  75                  80
   Pro Tyr Ile Thr Thr Trp Arg Ser Leu Ala Thr Asp Pro Pro Pro Trp
                   85                  90                  95
   Val Arg Pro Phe Leu Pro Pro Pro Lys His Pro Arg Thr Asp Pro Pro
               100                 105                 110
   Glu Pro Leu Ser Pro Gln Pro Leu Ala Pro Gln Pro Ser Ala Pro Pro
           115                 120                 125
   Ile Ser Ser Leu Tyr Pro Val Leu Pro Lys Pro Asp Pro Pro Lys Ala
       130                 135                 140
   Pro Val Leu Pro Pro Asn Pro Ser Ser Pro Leu Ile Asp Leu Leu Thr
   145                 150                 155                 160
   Glu Glu Pro Pro Pro Tyr Pro Gly Gly His Gly Pro Thr Pro Pro Ser
                   165                 170                 175
   Gly Pro Arg Thr Pro Thr Ala Ser Pro Ile Ala Ser Arg Leu Arg Glu
               180                 185                 190
   Arg Arg Glu Asn Pro Ala Glu Lys Ser Gln Ala Leu Pro Leu Arg Glu
           195                 200                 205
   Asp Pro Asn Asn Arg Pro Gln Tyr Trp Pro Phe Ser Ala Ser Asp Leu
       210                 215                 220
   Tyr Asn Trp Lys Leu His Asn Pro Pro Phe Ser Gln Asp Pro Val Ala
   225                 230                 235                 240
   Leu Thr Asn Leu Ile Glu Ser Ile Leu Val Thr His Gln Pro Thr Trp
                   245                 250                 255
   Asp Asp Cys Gln Gln Leu Leu Gln Ala Leu Leu Thr Ala Glu Glu Arg
               260                 265                 270
   Gln Arg Val Leu Leu Glu Ala Arg Lys Gln Val Pro Gly Glu Asp Gly
           275                 280                 285
   Arg Pro Thr Gln Leu Pro Asn Val Val Asp Glu Ala Phe Pro Leu Thr
       290                 295                 300
   Arg Pro Asn Trp Asp Phe Cys Thr Pro Ala Gly Arg Glu His Leu Arg
   305                 310                 315                 320
   Leu Tyr Arg Gln Leu Leu Leu Ala Gly Leu Arg Gly Ala Ala Arg Arg
                   325                 330                 335
   Pro Thr Asn Leu Ala Gln Val Lys Gln Val Val Gln Gly Lys Glu Glu
               340                 345                 350
   Thr Pro Ala Ser Phe Leu Glu Arg Leu Lys Glu Ala Tyr Arg Met Tyr
           355                 360                 365
   Thr Pro Tyr Asp Pro Glu Asp Pro Gly Gln Ala Thr Ser Val Ile Leu
       370                 375                 380
   Ser Phe Ile Tyr Gln Ser Ser Pro Asp Ile Arg Asn Lys Leu Gln Arg
   385                 390                 395                 400
   Leu Glu Gly Leu Gln Gly Phe Thr Leu Ser Asp Leu Leu Lys Glu Ala
                   405                 410                 415
   Glu Lys Ile Tyr Asn Lys Arg Glu Thr Pro Glu Glu Arg Glu Glu Arg
               420                 425                 430
   Leu Trp Gln Arg Gln Glu Glu Arg Asp Lys Lys Arg His Lys Glu Met
           435                 440                 445
   Thr Lys Val Leu Ala Thr Val Val Ala Gln Asn Arg Asp Lys Asp Arg
   450 455 460
   Gly Glu Ser Lys Leu Gly Asp Gln Arg Lys Ile Pro Leu Gly Lys Asp
   465 470 475 480
   Gln Cys Ala Tyr Cys Lys Glu Lys Gly His Trp Val Arg Asp Cys Pro
   485 490 495
   Lys Arg Pro Arg Lys Lys Pro Ala Asn Ser Thr Leu Leu
   500 505
<210> 7
   <211> 1929
   <212> DNA
   <213> Feline leukemia virus
   <220>
   <221> misc_feature
   <222> (1)..(1929)
   <223> DNA sequence for the mutagenized "env" gene (gp85)
<400> 7
   atggagtccc ccacccaccc caagccctcc aaggacaaga ccctgtcctg gaacatggtg 60
   ttcctggtgg gcatcctgtt caccattgac attggcatgg ccaacccctc ccccccccgg 120
   atctacaatg tgacctgggt gatcaccaat gtgcagacca acacccaggc caatgccacc 180
   tctatgctgg gcaccctgac agatgcatac cccaccctgc atgtggacct gtgtgacctg 240
   gtgggggaca cctgggagcc cattccgctg aaccccacca atgtgaagca tggggccagg 300
   tactcctcct ccaagtatgg ctgcaagacc acagacagga agaagcagca gcagacctac 360
   cccttctatg tgtgccctgg ccatgccccc tccctgggcc ccaagggcac ccactgtggg 420
   ggggcccagg atggcttctg tgctgcctgg ggctgtgaaa ccacagggga ggcctggtgg 480
   aagcccacct cctcctggga ctacatcaca gtgaagaggg gctcctccca ggacaactcc 540
   tgtgagggca agtgcaaccc cctggtgctg cagttcaccc agaagggcag gcaggcctcc 600
   tgggatggcc ccaagatgtg gggcctgagg ctgtacagga caggctatga ccccattgcc 660
   ctgttcacag tgtccaggca ggtgtccacc atcacccccc cccaggccat gggccccaac 720
   ctggtgctgc ctgaccagaa gcccccctcc aggcagtccc agacaggctc caaggtggcc 780
   acccagaggc cccagaccaa tgagtctgcc cccaggtctg tggcccccac caccatgggc 840
   cccaagagga ttggcacagg ggacaggctg atcaacctgg tgcagggcac ctacctggcc 900
   ctgaatgcca cagaccccaa caagaccaag gactgctggc tgtgcctggt gtccaggccc 960
   ccctactatg agggcattgc catcctgggc aactactcca accagaccaa cccccccccc 1020
   tcctgcctgt ccacccccca gcacaagctg accatctctg aggtgtctgg ccagggcatg 1080
   tgcattggca cagtgcccaa gacccaccag gccctgtgca acaagaccca gcagggccac 1140
   acaggggccc actacctggc tgtccccaat ggcacctact gggcctgcaa cacaggcctg 1200
   accccctgca tctccatggc tgtgctgaac tggacctctg acttctgtgt gctgattgag 1260
   ctgtggccca gggtgaccta ccaccagcct gagtatgtgt acacccactt tgccaaggct 1320
   gtgaggttca ggagggagcc catctccctg acagtggccc tgatgctggg gggcctgaca 1380
   gtggggggca ttgctgctgg ggtgggcaca ggcaccaagg ccctgctgga aacagcccag 1440
   ttcagacaac tacaaatggc catgcacaca gacatccagg ccctagaaga gtcagttagc 1500
   gctttagaaa aatccctgac ctccctctct gaagtagtcc tacaaaacag acgaggccta 1560
   gatattctat tcctacaaga gggaggactc tgtgccgcat taaaagaaga atgttgtttt 1620
   tatgcagatc acaccggatt agtccgagat aatatggcta aattaagaga aagattaaaa 1680
   cagcggcaac aactgtttga ctcccaacag ggatggtttg aaggatggtt caacaagtcc 1740
   ccctggctta caaccctaat ttcctctatt atgggcccct tgcttatcct gctcctaatt 1800
   ctcctcttcg gcccatgcat ccttaaccga ttggtgcaat tcgtaaaaga cagaatatcg 1860
   gtggtacaag ccttagtttt aacccaacag taccaacaga taaagcaata cgatccggac 1920
   cgaccatga 1929
<210> 8
   <211> 1440
   <212> DNA
   <213> Feline leukemia virus
<220>
   <221> misc_feature
   <222> (1)..(1440)
   <223> DNA Sequence of the mutagenized "env" gene (gp70)
<400> 8
   atggagtccc ccacccaccc caagccctcc aaggacaaga ccctgtcctg gaacatggtg 60
   ttcctggtgg gcatcctgtt caccattgac attggcatgg ccaacccctc ccccccccgg 120
   atctacaatg tgacctgggt gatcaccaat gtgcagacca acacccaggc caatgccacc 180
   tctatgctgg gcaccctgac agatgcatac cccaccctgc atgtggacct gtgtgacctg 240
   gtgggggaca cctgggagcc cattccgctg aaccccacca atgtgaagca tggggccagg 300
   tactcctcct ccaagtatgg ctgcaagacc acagacagga agaagcagca gcagacctac 360
   cccttctatg tgtgccctgg ccatgccccc tccctgggcc ccaagggcac ccactgtggg    420
   ggggcccagg atggcttctg tgctgcctgg ggctgtgaaa ccacagggga ggcctggtgg    480
   aagcccacct cctcctggga ctacatcaca gtgaagaggg gctcctccca ggacaactcc    540
   tgtgagggca agtgcaaccc cctggtgctg cagttcaccc agaagggcag gcaggcctcc    600
   tgggatggcc ccaagatgtg gggcctgagg ctgtacagga caggctatga ccccattgcc    660
   ctgttcacag tgtccaggca ggtgtccacc atcacccccc cccaggccat gggccccaac    720
   ctggtgctgc ctgaccagaa gcccccctcc aggcagtccc agacaggctc caaggtggcc    780
   acccagaggc cccagaccaa tgagtctgcc cccaggtctg tggcccccac caccatgggc    840
   cccaagagga ttggcacagg ggacaggctg atcaacctgg tgcagggcac ctacctggcc    900
   ctgaatgcca cagaccccaa caagaccaag gactgctggc tgtgcctggt gtccaggccc    960
   ccctactatg agggcattgc catcctgggc aactactcca accagaccaa cccccccccc   1020
   tcctgcctgt ccacccccca gcacaagctg accatctctg aggtgtctgg ccagggcatg  1080
   tgcattggca cagtgcccaa gacccaccag gccctgtgca acaagaccca gcagggccac   1140
   acaggggccc actacctggc tgtccccaat ggcacctact gggcctgcaa cacaggcctg   1200
   accccctgca tctccatggc tgtgctgaac tggacctctg acttctgtgt gctgattgag  1260
   ctgtggccca gggtgaccta ccaccagcct gagtatgtgt acacccactt tgccaaggct   1320
   gtgaggttca ggagggagcc catctccctg acagtggccc tgatgctggg gggcctgaca   1380
   gtggggggca ttgctgctgg ggtgggcaca ggcaccaagg ccctgctgga aacagcctga  1440
<210> 9
   <211> 642
   <212> PRT
   <213> Feline leukemia virus
<220>
   <221> PEPTIDE
   <222> (1)..(642)
   <223> Amino acid sequence of the protein corresponding to seq.ID7
<400> 9
   Met Glu Ser Pro Thr His Pro Lys Pro Ser Lys Asp Lys Thr Leu Ser
   1               5                   10                  15
   Trp Asn Met Val Phe Leu Val Gly Ile Leu Phe Thr Ile Asp Ile Gly
               20                  25                  30
   Met Ala Asn Pro Ser Pro Pro Arg Ile Tyr Asn Val Thr Trp Val Ile
           35                  40                  45
   Thr Asn Val Gln Thr Asn Thr Gln Ala Asn Ala Thr Ser Met Leu Gly
       50                  55                  60
   Thr Leu Thr Asp Ala Tyr Pro Thr Leu His Val Asp Leu Cys Asp Leu
   65                  70                  75                  80
   Val Gly Asp Thr Trp Glu Pro Ile Pro Leu Asn Pro Thr Asn Val Lys
                   85                  90                  95
   His Gly Ala Arg Tyr Ser Ser Ser Lys Tyr Gly Cys Lys Thr Thr Asp
               100                 105                 110
   Arg Lys Lys Gln Gln Gln Thr Tyr Pro Phe Tyr Val Cys Pro Gly His
           115                 120                 125
   Ala Pro Ser Leu Gly Pro Lys Gly Thr His Cys Gly Gly Ala Gln Asp
       130                 135                 140
   Gly Phe Cys Ala Ala Trp Gly Cys Glu Thr Thr Gly Glu Ala Trp Trp
   145                 150                 155                 160
   Lys Pro Thr Ser Ser Trp Asp Tyr Ile Thr Val Lys Arg Gly Ser Ser
                   165                 170                 175
   Gln Asp Asn Ser Cys Glu Gly Lys Cys Asn Pro Leu Val Leu Gln Phe
               180                 185                 190
   Thr Gln Lys Gly Arg Gln Ala Ser Trp Asp Gly Pro Lys Met Trp Gly
           195                 200                 205
   Leu Arg Leu Tyr Arg Thr Gly Tyr Asp Pro Ile Ala Leu Phe Thr Val
       210                 215                 220
   Ser Arg Gln Val Ser Thr Ile Thr Pro Pro Gln Ala Met Gly Pro Asn
   225                 230                 235                 240
   Leu Val Leu Pro Asp Gln Lys Pro Pro Ser Arg Gln Ser Gln Thr Gly
                   245                 250                 255
   Ser Lys Val Ala Thr Gln Arg Pro Gln Thr Asn Glu Ser Ala Pro Arg
               260                 265                 270
   Ser Val Ala Pro Thr Thr Met Gly Pro Lys Arg Ile Gly Thr Gly Asp
           275                 280                 285
   Arg Leu Ile Asn Leu Val Gln Gly Thr Tyr Leu Ala Leu Asn Ala Thr
       290                 295                 300
   Asp Pro Asn Lys Thr Lys Asp Cys Trp Leu Cys Leu Val Ser Arg Pro
   305 310 315 320
   Pro Tyr Tyr Glu Gly Ile Ala Ile Leu Gly Asn Tyr Ser Asn Gln Thr
   325 330 335
   Asn Pro Pro Pro Ser Cys Leu Ser Thr Pro Gln His Lys Leu Thr Ile
   340 345 350
   Ser Glu Val Ser Gly Gln Gly Met Cys Ile Gly Thr Val Pro Lys Thr
   355 360 365
   His Gln Ala Leu Cys Asn Lys Thr Gln Gln Gly His Thr Gly Ala His
   370 375 380
   Tyr Leu Ala Val Pro Asn Gly Thr Tyr Trp Ala Cys Asn Thr Gly Leu
   385 390 395 400
   Thr Pro Cys Ile Ser Met Ala Val Leu Asn Trp Thr Ser Asp Phe Cys
   405 410 415
   Val Leu Ile Glu Leu Trp Pro Arg Val Thr Tyr His Gln Pro Glu Tyr
   420 425 430
   Val Tyr Thr His Phe Ala Lys Ala Val Arg Phe Arg Arg Glu Pro Ile
   435 440 445
   Ser Leu Thr Val Ala Leu Met Leu Gly Gly Leu Thr Val Gly Gly Ile
   450 455 460
   Ala Ala Gly Val Gly Thr Gly Thr Lys Ala Leu Leu Glu Thr Ala Gln
   465 470 475 480
   Phe Arg Gln Leu Gln Met Ala Met His Thr Asp Ile Gln Ala Leu Glu
   485 490 495
   Glu Ser Val Ser Ala Leu Glu Lys Ser Leu Thr Ser Leu Ser Glu Val
   500 505 510
   Val Leu Gln Asn Arg Arg Gly Leu Asp Ile Leu Phe Leu Gln Glu Gly
   515 520 525
   Gly Leu Cys Ala Ala Leu Lys Glu Glu Cys Cys Phe Tyr Ala Asp His
   530 535 540
   Thr Gly Leu Val Arg Asp Asn Met Ala Lys Leu Arg Glu Arg Leu Lys
   545 550 555 560
   Gln Arg Gln Gln Leu Phe Asp Ser Gln Gln Gly Trp Phe Glu Gly Trp
   565 570 575
   Phe Asn Lys Ser Pro Trp Leu Thr Thr Leu Ile Ser Ser Ile Met Gly
   580 585 590
   Pro Leu Leu Ile Leu Leu Leu Ile Leu Leu Phe Gly Pro Cys Ile Leu
   595 600 605
   Asn Arg Leu Val Gln Phe Val Lys Asp Arg Ile Ser Val Val Gln Ala
   610 615 620
   Leu Val Leu Thr Gln Gln Tyr Gln Gln Ile Lys Gln Tyr Asp Pro Asp
   625 630 635 640
   Arg Pro
<210> 10
   <211> 479
   <212> PRT
   <213> Feline leukemia virus
<220>
   <221> PEPTIDE
   <222> (1)..(479)
   <223> Amino acid sequence of the protein corresponding to Seq.ID8
<400> 10
   Met Glu Ser Pro Thr His Pro Lys Pro Ser Lys Asp Lys Thr Leu Ser
   1 5 10 15
   Trp Asn Met Val Phe Leu Val Gly Ile Leu Phe Thr Ile Asp Ile Gly
   20 25 30
   Met Ala Asn Pro Ser Pro Pro Arg Ile Tyr Asn Val Thr Trp Val Ile
   35 40 45
   Thr Asn Val Gln Thr Asn Thr Gln Ala Asn Ala Thr Ser Met Leu Gly
   50 55 60
   Thr Leu Thr Asp Ala Tyr Pro Thr Leu His Val Asp Leu Cys Asp Leu
   65 70 75 80
   Val Gly Asp Thr Trp Glu Pro Ile Pro Leu Asn Pro Thr Asn Val Lys
   85 90 95
   His Gly Ala Arg Tyr Ser Ser Ser Lys Tyr Gly Cys Lys Thr Thr Asp
   100 105 110
   Arg Lys Lys Gln Gln Gln Thr Tyr Pro Phe Tyr Val Cys Pro Gly His
           115                 120                 125
   Ala Pro Ser Leu Gly Pro Lys Gly Thr His Cys Gly Gly Ala Gln Asp
       130                 135                 140
   Gly Phe Cys Ala Ala Trp Gly Cys Glu Thr Thr Gly Glu Ala Trp Trp
   145                 150                 155                 160
   Lys Pro Thr Ser Ser Trp Asp Tyr Ile Thr Val Lys Arg Gly Ser Ser
                   165                 170                 175
   Gln Asp Asn Ser Cys Glu Gly Lys Cys Asn Pro Leu Val Leu Gln Phe
               180                 185                 190
   Thr Gln Lys Gly Arg Gln Ala Ser Trp Asp Gly Pro Lys Met Trp Gly
           195                 200                 205
   Leu Arg Leu Tyr Arg Thr Gly Tyr Asp Pro Ile Ala Leu Phe Thr Val
       210                 215                 220
   Ser Arg Gln Val Ser Thr Ile Thr Pro Pro Gln Ala Met Gly Pro Asn
   225                 230                 235                 240
   Leu Val Leu Pro Asp Gln Lys Pro Pro Ser Arg Gln Ser Gln Thr Gly
                   245                 250                 255
   Ser Lys Val Ala Thr Gln Arg Pro Gln Thr Asn Glu Ser Ala Pro Arg
               260                 265                 270
   Ser Val Ala Pro Thr Thr Met Gly Pro Lys Arg Ile Gly Thr Gly Asp
           275                 280                 285
   Arg Leu Ile Asn Leu Val Gln Gly Thr Tyr Leu Ala Leu Asn Ala Thr
       290                 295                 300
   Asp Pro Asn Lys Thr Lys Asp Cys Trp Leu Cys Leu Val Ser Arg Pro
   305                 310                 315                 320
   Pro Tyr Tyr Glu Gly Ile Ala Ile Leu Gly Asn Tyr Ser Asn Gln Thr
                   325                 330                 335
   Asn Pro Pro Pro Ser Cys Leu Ser Thr Pro Gln His Lys Leu Thr Ile
               340                 345                 350
   Ser Glu Val Ser Gly Gln Gly Met Cys Ile Gly Thr Val Pro Lys Thr
           355                 360                 365
   His Gln Ala Leu Cys Asn Lys Thr Gln Gln Gly His Thr Gly Ala His
       370                 375                 380
   Tyr Leu Ala Val Pro Asn Gly Thr Tyr Trp Ala Cys Asn Thr Gly Leu
   385 390 395 400
   Thr Pro Cys Ile Ser Met Ala Val Leu Asn Trp Thr Ser Asp Phe Cys
   405 410 415
   Val Leu Ile Glu Leu Trp Pro Arg Val Thr Tyr His Gln Pro Glu Tyr
   420 425 430
   Val Tyr Thr His Phe Ala Lys Ala Val Arg Phe Arg Arg Glu Pro Ile
   435 440 445
   Ser Leu Thr Val Ala Leu Met Leu Gly Gly Leu Thr Val Gly Gly Ile
   450 455 460
   Ala Ala Gly Val Gly Thr Gly Thr Lys Ala Leu Leu Glu Thr Ala
   465 470 475
<210> 11
   <211> 1440
   <212> DNA
   <213> Feline leukemia virus
<220>
   <221> gene
   <222> (1)..(1440)
   <223> DNA sequence of wildtype "env" gene (gp70)
<400> 11
   atggaaagtc caacgcaccc aaaaccctct aaagataaga ctctctcgtg gaacttagcg    60
   tttctggtgg ggatcttatt tacaatagac ataggaatgg ccaatcctag tccacaccaa   120
   atatataatg taacttgggt aataaccaat gtacaaacta acacccaagc taacgccacc   180
   tctatgttag gaaccttaac cgatgcctac cctaccctac atgttgactt atgtgaccta   240
   gtgggagaca cctgggaacc tatagtccta aacccaacca atgtaaaaca cggggcacgt   300
   tactcctcct caaaatatgg atgtaaaact acagatagaa aaaaacagca acagacatac   360
   cccttttacg tctgccccgg acatgccccc tcgttggggc caaagggaac acattgtgga    420
   ggggcacaag atgggttttg tgccgcatgg ggatgtgaga ccaccggaga agcttggtgg   480
   aagcccacct cctcatggga ctatatcaca gtaaaaagag ggagtagtca ggacaatagc   540
   tgtgagggaa aatgcaaccc cctggttttg cagttcaccc agaagggaag acaagcctct   600
   tgggacggac ctaagatgtg gggattgcga ctataccgta caggatatga ccctatcgct   660
   ttattcacgg tgtcccggca ggtatcaacc attacgccgc ctcaggcaat gggaccaaac     720
   ctagtcttac ctgatcaaaa acccccatcc cgacaatctc aaacagggtc caaagtggcg     780
   acccagaggc cccaaacgaa tgaaagcgcc ccaaggtctg ttgcccccac caccatgggt     840
   cccaaacgga ttgggaccgg agataggtta ataaatttag tacaagggac atacctagcc     900
   ttaaatgcca ccgaccccaa caaaactaaa gactgttggc tctgcctggt ttctcgacca     960
   ccctattacg aagggattgc aatcttaggt aactacagca accaaacaaa ccccccccca     1020
   tcctgcctat ctactccgca acacaaacta actatatctg aagtatcagg gcaaggaatg    1080
   tgcataggga ctgttcctaa aacccaccag gctttgtgca ataagacaca acagggacat    1140
   acaggggcgc actatctagc cgcccccaac ggcacctatt gggcctgtaa cactggactc    1200
   accccatgca tttccatggc ggtgctcaat tggacctctg atttttgtgt cttaatcgaa    1260
   ttatggccca gagtgactta ccatcaaccc gaatatgtgt acacacattt tgccaaagct    1320
   gtcaggttcc gaagagaacc aatatcacta acggttgccc ttatgttggg aggacttact    1380
   gtagggggca tagccgcggg ggtcggaaca gggactaaag ccctccttga aacagcctga    1440
<210> 12
   <211> 42
   <212> DNA
   <213> Primer
<220>
   <221> misc_feature
   <222> (1)..(42)
   <223> gag-mut1-rneu
<400> 12
   aattaagagc tccacgtctc cccccgctaa cagcaactgg cg 42
<210> 13
   <211> 45
   <212> DNA
   <213> Primer
<220>
   <221> misc_feature
   <222> (1)..(45)
   <223> gag-mut2-1
<400> 13
   aattaagagc tccaggtctc cggggctccg cggggctgca agacg 45
<210> 14
   <211> 48
   <212> DNA
   <213> Primer
<220>
   <221> misc_feature
   <222> (1)..(48)
   <223> gag-mut3-r
<400> 14
   aattaagagc tccacgtctc cttccctttt gttgtatatc ttttctgc 48
<210> 15
   <211> 48
   <212> DNA
   <213> Primer
<220>
   <221> misc_feature
   <222> (1)..(48)
   <223> gag-mut4-1
<400> 15
   aattaagagc tccaggtctc cggaaacccc agaggaaagg gaagaaag 48
<210> 16
   <211> 34
   <212> DNA
   <213> Primer
<220>
   <221> mise_ feature
   <222> (1)..(34)
   <223> Felvgag-1
<400> 16
   cggataaggt accatgggcc aaactataac tacc 34
<210> 17
   <211> 37
   <212> DNA
   <213> Primer
<220>
   <221> misc_feature
   <222> (1)..(37)
   <223> Felvgag-r
<400> 17
   ttctcagagc tcttagagga gagtggagtt ggcgggt 37
<210> 18
   <211> 33
   <212> DNA
   <213> Primer
<220>
   <221> misc_feature
   <222> (1)..(33)
   <223> envl
<400> 18
   cggataaggt accatggcca atcctagtcc acc 33
<210> 19
   <211> 37
   <212> DNA
   <213> Primer
<220>
   <221> misc_feature
   <222> (1)..(37)
   <223> envr
<400> 19
   agttctcaga gctcttaggc tgtttcaagg agggctt 37
<210> 20
   <211> 28
   <212> DNA
   <213> Primer
<220>
   <221> mise_feature
   <222> (1)..(28)
   <223> Primer
<400> 20
   atattggatc ccatggccaa cccctccc 28
<210> 21
   <211> 34
   <212> DNA
   <213> Primer
<220>
   <221> misc_feature
   <222> (1)..(34)
   <223> Primer
<400> 21
   attatggtct cctgctgctt cttcctgtct gtgg 34
<210> 22
   <211> 30
   <212> DNA
   <213> Primer
<220>
   <221> misc_feature
   <222> (1)..(30)
   <223> Primer
<400> 22
   taataggtct ccagcagcag acctacccct 30
<210> 23
   <211> 33
   <212> DNA
   <213> Primer
<220>
   <221> misc_feature
   <222> (1)..(33)
   <223> Primer
<400> 23
   taataggtct ctgtgaacag ggcaatgggg tca 33
<210> 24
   <211> 34
   <212> DNA
   <213> Primer
<220>
   <221> misc_feature
   <222> (1)..(34)
   <223> Primer
<400> 24
   tatttggtct cttcacagtg tccaggcagg tgtc 34
<210> 25
   <211> 30
   <212> DNA
   <213> Primer
<220>
   <221> misc_feature
   <222> (1)..(30)
   <223> Primer
<400> 25
   tattaggtct cagcttgtgc tggggggtgg 30
<210> 26
   <211> 34
   <212> DNA
   <213> Primer
<220>
   <221> misc_feature
   <222> (1)..(34)
   <223> Primer
<400> 26
   aataaggtct ccaagctgac catctctgag gtgt 34
<210> 27
   <211> 27
   <212> DNA
   <213> Primer
<220>
   <221> misc_feature
   <222> (1)..(27)
   <223> Primer
<400> 27
   attaagagct ctcaggctgt ttccagc 27
<210> 28
   <211> 31
   <212> DNA
   <213> Primer
<220>
   <221> misc_feature
   <222> (1)..(31)
   <223> Primer
<400> 28
   attgccggta ccatggagtc ccccacccac c 31
<210> 29
   <211> 35
   <212> DNA
   <213> Primer
<220>
   <221> misc_feature
   <222> (1)..(35)
   <223> Primer
<400> 29
   atcagaggtc tcccatgcca atgtcaatgg tgaac 35
<210> 30
   <211> 27
   <212> DNA
   <213> Primer
<220>
   <221> misc_feature
   <222> (1)..(27)
   <223> Primer
<400> 30
   gatctgggtc tccatggcca acccctc 27
<210> 31
   <211> 36
   <212> DNA
   <213> Primer
<220>
   <221> misc_feature
   <222> (1)..(36)
   <223> Primer
<400> 31
   aattatggtc tcgcagttca gacaactaca aatggc 36
<210> 32
   <211> 30
   <212> DNA
   <213> Primer
<220>
   <221> misc_feature
   <222> (1)..(30)
   <223> Primer
<400> 32
   aattatgagc tctcagggcc tgtcagggtc 30
<210> 33
   <211> 28
   <212> DNA
   <213> Primer
<220>
   <221> misc_feature
   <222> (1)..(28)
   <223> Primer
<400> 33
   aattatggta ccatggagtc ccccaccc 28
<210> 34
   <211> 35
   <212> DNA
   <213> Primer
<220>
   <221> misc_feature
   <222> (1)..(35)
   <223> Primer
<400> 34
   tataatggtc tcaactgggc tgtttccagc agggc 35
<210> 35
   <211> 31
   <212> DNA
   <213> Primer
<220>
   <221> misc_feature
   <222> (1)..(31)
   <223> Primer
<400> 35
   atattaggtc tcagatccgg gggggggagg g 31
<210> 36
   <211> 30
   <212> DNA
   <213> Primer
<220>
   <221> misc_feature
   <222> (1)..(30)
   <223> Primer
<400> 36
   atattggtct caggagaggg acaagaagag 30
<210> 37
   <211> 32
   <212> DNA
   <213> Primer
<220>
   <221> misc_feature
   <222> (1)..(32)
   <223> Primer
<400> 37
   aatatggtct ctcagcctgc tggcgatggg gc 32
<210> 38
   <211> 32
   <212> DNA
   <213> Primer
<220>
   <221> misc_feature
   <222> (1)..(32)
   <223> Primer
<400> 38
   attatggtct ctgcacctga ggctgtacag gc 32
<210> 39
   <211> 36
   <212> DNA
   <213> Primer
<220>
   <221> misc_feature
   <222> (1)..(36)
   <223> Primer
<400> 39
   aatatggtct cggtgctccc tgccggcggg ggtgca 36
<210> 40
   <211> 28
   <212> DNA
   <213> Primer
<220>
   <221> misc_feature
   <222> (1)..(28)
   <223> Primer
<400> 40
   aatatggtct ctctcctcct gcctctgc 28